# EUROPEAN PATENT APPLICATION

(11) **EP 1 905 836 A1**
(43) Date of publication of application: **02.04.2008**
(21) Application number: 06291528.5
(22) Date of filing: 28.09.2006
(51) Int. Cl.: C12N 15/81, C07K 14/56

(54) **Recombinant Pichia pastoris strains and process for the production of recombinant human interferon alpha**

(71) Applicant: INSTITUT PASTEUR, 75015 Paris (FR); INSTITUT PASTEUR DE TUNIS, 1002 Tunis (TN)
(72) Inventor: Fathallah, Mohamed Dahmani, 2092 Tunis (TN); Kallel, Héla, 20092 El Menzeh (TN); Dellagi, Koussay, 1002 Tunis Bélvédère (TN); Rebhi, Imen, 2033 Mégrine côteaux (TN); Ayed, Atef, 2036 Soukra (TN)
(74) Representative: Marcadé, Véronique

(57) **Abstract**

The present invention relates to the field of production of recombinant human interferon alpha. More specifically, the invention discloses novel recombinant *Pichia pastoris* strains that have been genetically engineered for efficiently producing two kinds of active recombinant human interferon alpha: human interferon alpha 2a and human interferon alpha 2b. The invention also pertains to optimized processes for producing and purifying said interferon alpha.

## Description

The present invention relates to the field of production of recombinant human interferon alpha. More specifically, the invention discloses novel recombinant *Pichia pastoris* strains that have been genetically engineered for efficiently producing two kinds of active recombinant human interferon alpha: human interferon alpha 2a and human interferon alpha 2b. The invention also pertains to optimized processes for producing and purifying said interferon alpha.

Interferons (IFNs) are essential components of the innate line of defence of vertebrates against infectious agents and tumour development and progression. Recombinant interferon α has been used for almost two decades, as a therapeutic agent in a variety of diseases ranging from viral infections to different types of cancer.

Recombinant human interferons have been expressed in *Escherichia coli* (Nagata et al., 1980; Remaut et al., 1983; Mizoguchi et al., 1985; Babu et al., 2000), yeast (Hitzeman et al., 1981; Zsebo et al., 1986), baculovirus infected insect cells (Smith et al., 1983; Voss et al., 1993) and animal cells (Rossmann et al., 1993). The yield of recombinant IFNs using *E. coli* is by far higher than the one obtained using the other systems. However, IFNs are produced in *E. coli* as insoluble inclusion bodies that need to be subjected to solubilization and refolding steps to allow generation of functional and active proteins (Babu et al., 2000). Such steps are usually time consuming and need extensive optimisation to reach reasonable production yields. Furthermore the products generated in *E. coli* and intended for human use need to be endotoxin free. This requirement further complicates the production processes using *E. coli* and impact negatively on the final cost of the products.

Yeast and particularly the methylotrophic yeast *Pichia pastoris,* have numerous advantages as host system for the production of recombinant proteins from higher eukaryotes. They combine the ease, simplicity and cheapness of bacterial expression systems. Yeast is simple to cultivate on inexpensive growth media and the array of techniques for the making of recombinant clones is now well advanced (Clare and Romanos, 1995; Sreekrishna et al., 1997; Cereghino and Cregg, 2000). Like eukaryotes, yeasts provide an adequate environment for post-translational processing and secretion, resulting in a product that is often identical or more similar to the native protein. Moreover, *Pichia pastoris* grows at very high cell density on minimal medium. The success of the *Pichia* system is largely due to the use of the strong, tightly regulated alcohol oxidase (AOX1) promoter to drive heterologous protein expression. In wild-type *Pichia,* this promoter controls the expression of alcohol oxidase 1, the first enzyme in methanol metabolism (Cregg et al., 1989b), which can account for up to 30% of the total soluble protein (Couderc and Baratti, 1980). Another key feature of the system is that a high cell density can be achieved using a minimal salt medium which is cost effective (Sreekrishna et al., 1989). The strong promoter, coupled with the high cell density fermentation, has allowed production of recombinant proteins at high concentration. The highest yields reported are 22 g per L of S-hydroxynitrile lyase, an intracellulary expressed enzyme (Hasslacher et al., 1997), and 11 g per L culture volume of secreted gelatine (Werten et al., 1999).

For these reasons, Sudhir et al. (WO 01/68827) have proposed a process for the production of human alpha interferon from genetically engineered *P. pastoris.* More recently, Villarete et al. (US 2003/0065148) proposed some improvements to this process, applied to Interferon a-1 (also known as IFNaD). In particular, they introduced, in the expression vector, fused to the 5' extremity of the IFNaD coding sequence, a sequence coding for a signal peptide sequence enabling the secretion of the recombinant protein. Even more recently, Lohray et al. (WO 2004/039996) described the production of IFNa2b using genetically-engineered *P. pastoris* strains transformed with an expression vector expressing a secreted IFNa2b. The producing strain and the process described in this document are however not optimal, since a complex medium is needed to obtain high production yields.

When using *P. pastoris* as a producing strain, different phenotypes of the recombinant clones can be obtained regarding methanol metabolism, depending on how the expression cassette is integrated into *Pichia* genome by homologous recombination. Mut⁺ clones can metabolise methanol quickly since in these clones the AOX1 gene is intact, whereas clones in which AOX1 gene has been replaced by the expression cassette due to a double cross-over event, show slow growth on methanol (Mut^{s}).

The Mut phenotype of the recombinant clone is important because protein expression driven by the AOX1 promoter requires the presence of methanol. Special care must be taken to keep the methanol concentration below toxic levels while maintaining the induction (Stratton et al., 1998).

Downstream processing is another key issue that needs to be considered during the design of biotechnological processes. Common purification processes of recombinant proteins employ sequential chromatography techniques such as cation exchange, anion exchange, gel filtration, etc. They are therefore costly and time-consuming.

The present invention aims at overcoming at least some of the limitations of the current processes for producing human alpha interferons.

A first aspect of the present invention is a nucleic acid molecule comprising a coding sequence encoding a human alpha interferon, wherein said sequence is optimized for expression in *Pichia pastoris.* Indeed, it has been shown that organisms display a non-random pattern of synonymous codon usage (Grantham et al., 1980; Nakamura et al., 1999), and that exogenous coding sequences using the codons which are more frequently used by the host itself may be more efficiently translated than sequences not respecting this codon bias. Hence, an "optimized sequence" for expression in *Pichia pastoris* is an artificial coding sequence in which the codons have been chosen according to the codon bias of *Pichia pastoris,* as disclosed for example by Sinclair et al. (2002).

In a particular embodiment of the nucleic acid according to the invention, the encoded human alpha interferon is HuIFNa2a and the coding sequence is SEQ ID No: 1.

Another nucleic acid according to the invention comprises a coding sequence encoding a human interferon a2b, wherein said coding sequence is SEQ ID No: 3. This sequence has been cloned by an RT-PCR approach, from healthy individual leukocytes exposed to the New Castle Disease virus.

The invention also relates to an expression vector for transforming a *Pichia pastoris* strain, which comprises, in the 5' to 3' direction and operably linked :
(i) a *Pichia pastoris*-recognized transcription and translation initiation region,
(ii) a sequence coding for a secretion signal enabling protein secretion in *Pichia pastoris,*
(iii) a coding sequence encoding a human alpha interferon, such as the sequences defined above (in particular, the sequences SEQ ID No: 1 or SEQ ID No: 3, or an optimized sequence for expression in *P. pastoris,* encoding a HuIFNa2b of SEQ ID No: 4), and
(iv) a *Pichia pastoris-*recognized transcription and translation termination region.

A *Pichia pastoris* strain which has been transformed by a vector as above-described, and which produces human alpha interferon, is also part of the present invention. In a preferred embodiment of this aspect of the invention, the transformed strain has been obtained by transforming a Mut^{S} KM71H *Pichia pastoris* strain.

Preferred examples of *Pichia pastoris* strains according to the invention are KM71H/PICZαrHuIFNα2b, deposited at the *Collection Nationale de Cultures de Microorganismes* (*CNCM*) on February 01, 2006, under the n°I-3562, and IRDF/INF/IPT05, deposited at the *Collection Nationale de Cultures de Microorganismes* (*CNCM*) on September 11, 2006, under the n°I-3668.

Another aspect of the present invention is a process for producing a human alpha interferon by culturing a *Pichia pastoris* strain which expresses, under the control of a promoter regulated by methanol, a secretable form of said human alpha interferon. This process comprises the following steps:
(i) inoculation of a synthetic culture medium in a bioreactor, by a preculture of said *Pichia pastoris* strain, wherein said synthetic medium contains glycerol as a carbon source, and has a defined salt composition;
(ii) batch culture in said bioreactor, at 30°C, pH5, under agitation and oxygenation;
(iii) fed-batch culture on glycerol at 30°C, pH5, under agitation and oxygenation;
(iv) induction phase: fed-batch culture on methanol, in a synthetic culture medium supplemented with EDTA at a final concentration of 10 mM.

Steps (i) to (iii) represent a biomass build-up phase, resulting in a great amount of *P. pastoris* cells, whereas step (iv) is an induction phase, during which said cells produce the interferon.

The process of the invention is preferably carried out with a *Pichia pastoris* strain according to the invention, as described above.

In the above process, the synthetic culture medium is preferably obtained as follows:
- glycerol (40 g/l), K₂SO₄ (18.2 g/l), MgSO₄ (7.28 g/l), KOH (4.13 g/l), CaSO₄.2H₂O (0.93 g/l) and 85% orthophosphoric acid (26,7 ml/l) are dissolved in deionised water and sterilized in the bioreactor;
- 5 ml/l basal salts of fermentation PTM1 and 2 ml/l of 500X biotin are added to the medium after sterilization, and culture medium pH is adjusted to 5 by addition of 25% ammonia (w/v), wherein the PTM1 solution contains: CuSO₄.5H₂O (6 g l⁻¹), NaI (0.08 g l⁻¹), MnSO₄.H₂O (3 g l⁻¹), Na₂MoO₄.2H₂O (0.2 g l⁻¹), H₃BO₃ (0.02 g l⁻¹), CoCl₂ (0.5 g l⁻¹), ZnCl₂ (20 g l⁻¹), FeSO₄.7H₂O (65 g l⁻¹), biotin (0.2 g l⁻¹) and H₂SO₄ 98% (5 ml l⁻¹).

When performing this process, the fed-batch solution used in step (iii) preferably contains glycerol (450 g/l), PTM1 (8 ml/l) and 500X biotin (5 ml/l), and is added at a rate of 15 ml/l/h.

The fed-batch solution used in the induction phase (step (iv) of the above-described process) preferably contains methanol at 100% (973 ml/1), 500X biotin (5 ml/1) and PTM1 (8 ml/1) and the feeding rate is modulated to keep the methanol residual level under 0.6% (v/v). The induction phase lasts for at least three days, preferably 4 or 5 days, during which the following components are added to the bioreactor at regular intervals of 24 hours: casaminoacids at a final concentration of 0.1% (w/v) and Yeast Nitrogen Base at a final concentration of 0.1 % (w/v).

In a particular way of performing the above process according to the invention, step (iii) is initiated once the optical density at 600 nm of the culture in step (ii) has reached 80, and the fed-batch culture of step (iii) lasts until the dry weight reaches at least 100 g/l, preferably 110-120 g/l.

In addition, it can be advantageous, when performing the production process according to the invention, to change the culture medium between the biomass build-up and the induction phases. In this case, the cells are harvested, resuspended in fresh medium and returned back to the bioreactor, between steps (iii) and (iv). Hence, the induction phase takes place in a medium which is completely free of complex medium, since the small volume of medium of the preculture is removed before the beginning of the induction phase. Indeed, it can be noted that the preculture used in step (i) is not necessarily done in a synthetic medium. The induction medium is preferably supplemented with EDTA (final concentration of 10 mM).

An example of detailed conditions for performing the production process according to the invention is described in Example 7 below. In this non-limiting example, the bioreactor is a 5-liter reactor, equipped with a process control system of data, and in which the batch culture is carried out with an initial culture volume of 2 litres inoculated with the 200 ml preculture in BMGY medium, in the following conditions: temperature = 30°C, stirrer speed = 800 rpm, pH maintained at 5 by addition of 25% ammonia, dissolved oxygen set at 40% of air saturation by stirrer cascading and enrichment of the inlet air with pure oxygen when required. The conditions of stirring, pH, and oxygenation are preferably the same during the induction phase.

Other aspects of the present invention are processes for purifying human alpha interferon secreted in the culture medium by recombinant yeasts. A first purification process according to the present invention comprises the following steps:
(i) eliminating the biomass by centrifugation,
(ii) filtering the supernatant through a 0.1 µm cartridge in the presence of Triton X-100 at 1%,
(iii) desalting the permeate by diafiltration using a 5 kDa cartridge and, for example, lactic acid 50 mM pH4,
(iv) purifying the supernatant by a cation exchange chromatography, for example on a Sepharose SP column, as disclosed in Example 8.1 below.

Alternatively, the desalting step (iii) can be performed by chromatography on a Sephadex G25 column.

A second process according to the invention, for purifying human alpha interferon secreted in the culture medium by recombinant yeasts, comprises the following steps:
(i) dilution of the culture medium with a solution of lactic acid (25 mM), NaCl (0.1 M), pH4, to an optical density at 600 nm equal to 100;
(ii) purification of the diluted medium using an expanded bed chromatography on a cation exchange support, for example a Streamline SP XL device as described in example 8.2 below.

When performing any of the purification processes according to the invention, a further step of purification by gel filtration, using sephacryl S-100, can be added.

Of course, these purification processes are preferably performed on medium obtained at the end of the induction phase (iv) of the production process described above, and/or on culture medium obtained after said human alpha interferon has been produced by a recombinant *Pichia pastoris* strain according to the present invention.

The invention is further illustrated by the following figures and examples.

### Legends to the figures

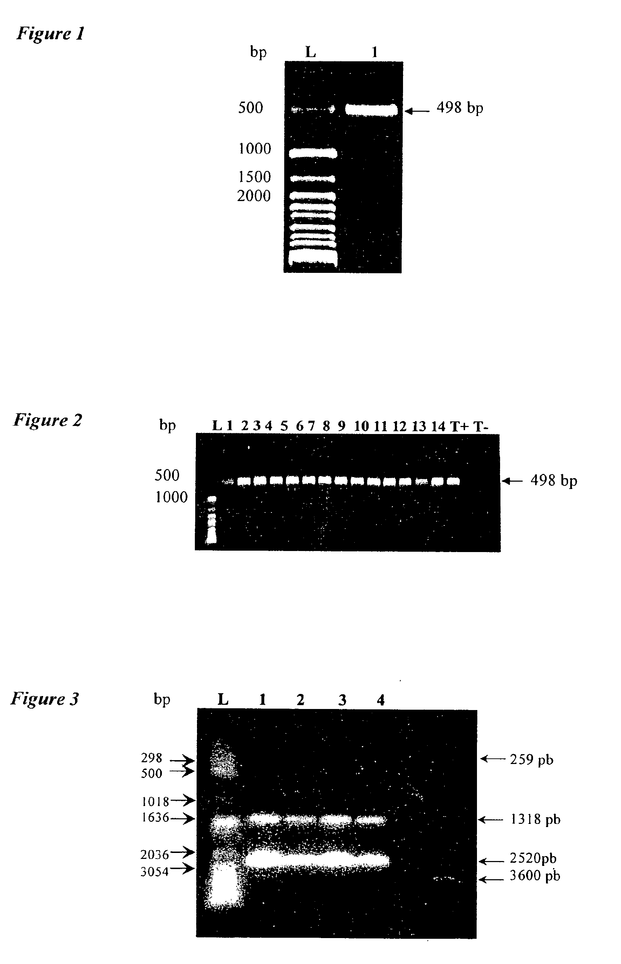

Figure 1: Lane 1 shows the 498 bp RT-PCR reaction product from *HuIFN-α2b* cDNA using the 5' EcoRI forward primer: FIFNα2b and the 3' STOP/NotI reverse primer: RIFNα2b. Aliquots of 5µl were electrophoresed on a 0.7% agarose gel, TBE 1X with the 1Kb DNA ladder (lane L).

Figure 2: Isolation of pPICZαA/rHuIFNα2b recombinant plasmid containing the cDNA sequence encoding human IFNα2b by "colonies-PCR" based method. Clones containing the 498 bp IFNα2b gene (Lane 2, 3, 4, 5, 6, 7, 8, 9 10, 11, 12 and 14) as well as lane T+ (Amplified IFNα2b product), show a 498 bp band. Lane T-corresponds to the mixed PCR solution.

Figure 3: Isolation of pPICZαA/rHuIFNα2b recombinant plasmid containing the cDNA sequence encoding human IFNα2b by restriction analysis using BglII restriction enzyme. Lane 1, 2, 3 and 4 represent respectively the clones 2, 4, 9 and 12 containing the IFNα2b gene, while lane 5 corresponds to the non recombinant pPICZαA plasmid (empty). 20µl Aliquots of *Bgl*II/digested products were electrophoresed on a 0.7% agarose gel, TBE 1X with the DNA markers (Boehringer Manheim, Germany) (lane L).

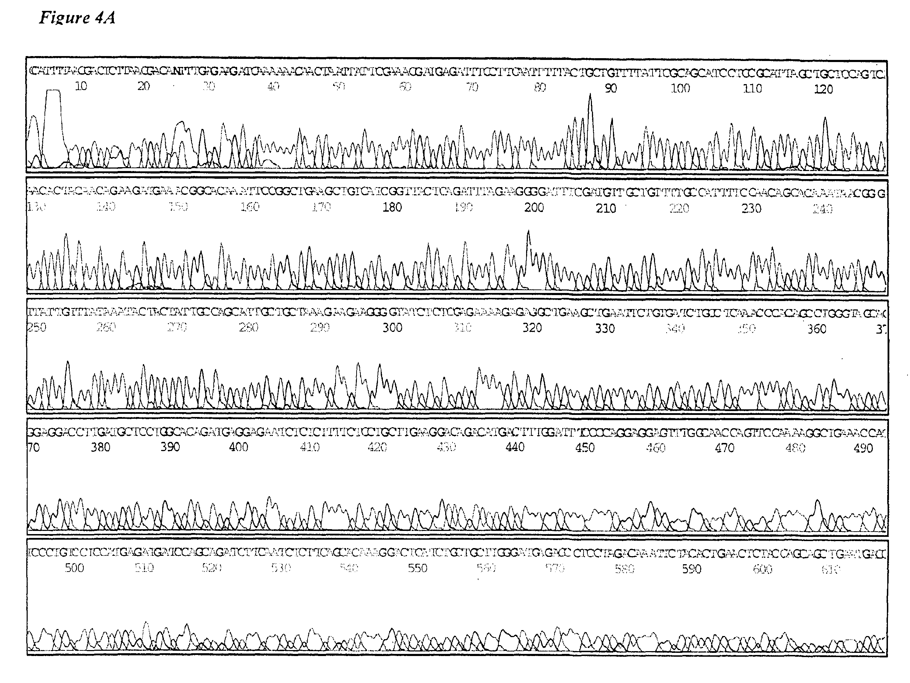

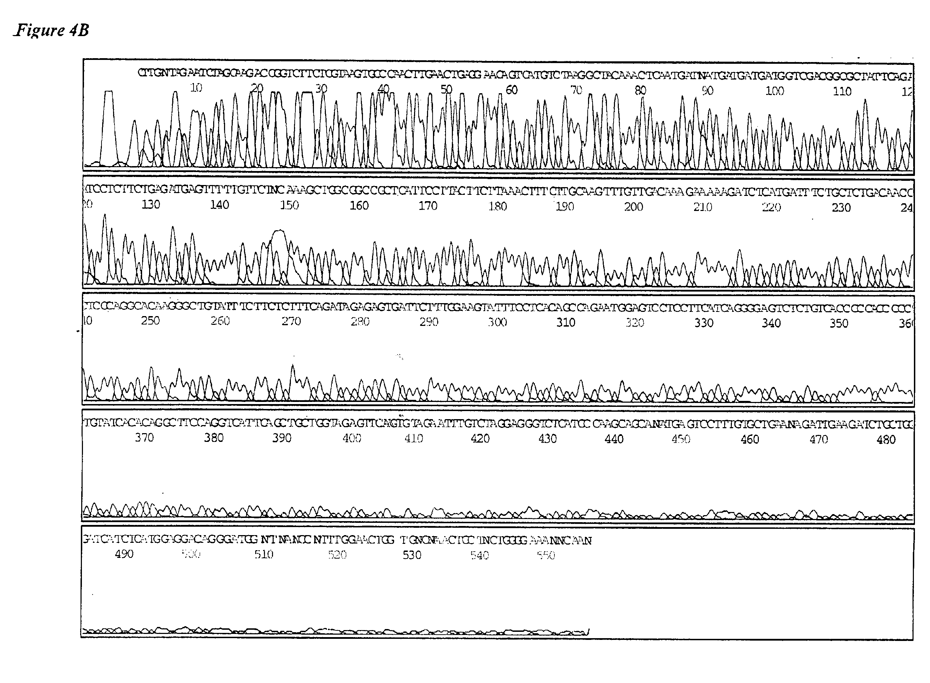

Figure 4: **Fig. 4A:** pPICZαA/rHuIFNα2b recombinant plasmid nucleotide sequence. Sequencing result obtained using the forward Aox1/F sequencing primer. **Fig. 4B:** pPICZαA/rHuIFNα2b recombinant plasmid nucleotide sequence. Sequencing Result obtained using the reverse Aox1/R sequencing primer.

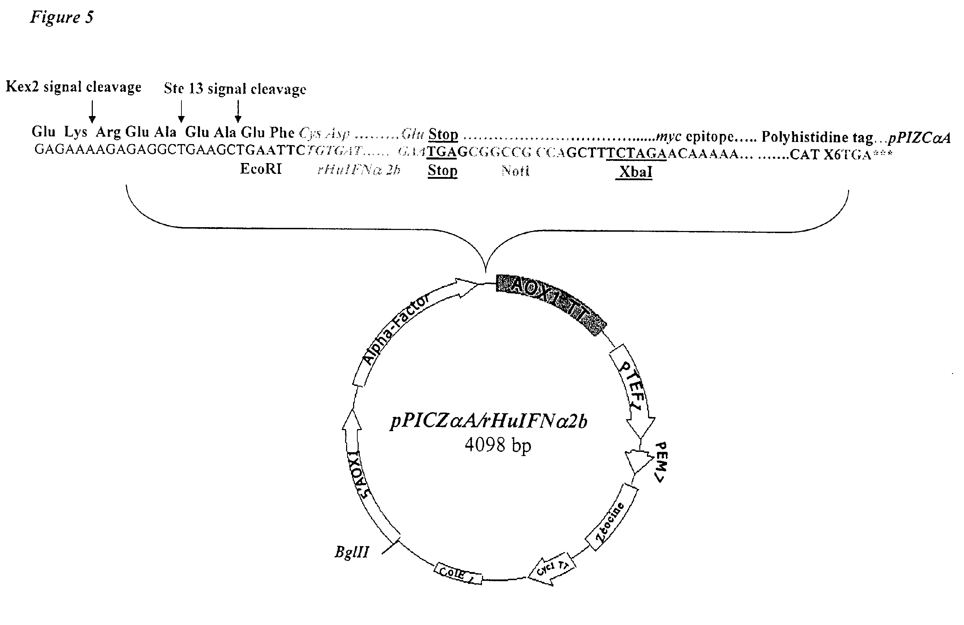

Figure 5: Map of the pPICZαA/rHuIFNα2b P.pastoris expression plasmid. The *rHu*IFN*α2b* gene was inserted between the 5' EcoRI and the 3'NotI restriction site. The TGA stop codon was introduced at the 3' end of *rHu*IFN*α2b* gene, to produce a native C-terminal *rHu*IFN*α2b* protein.

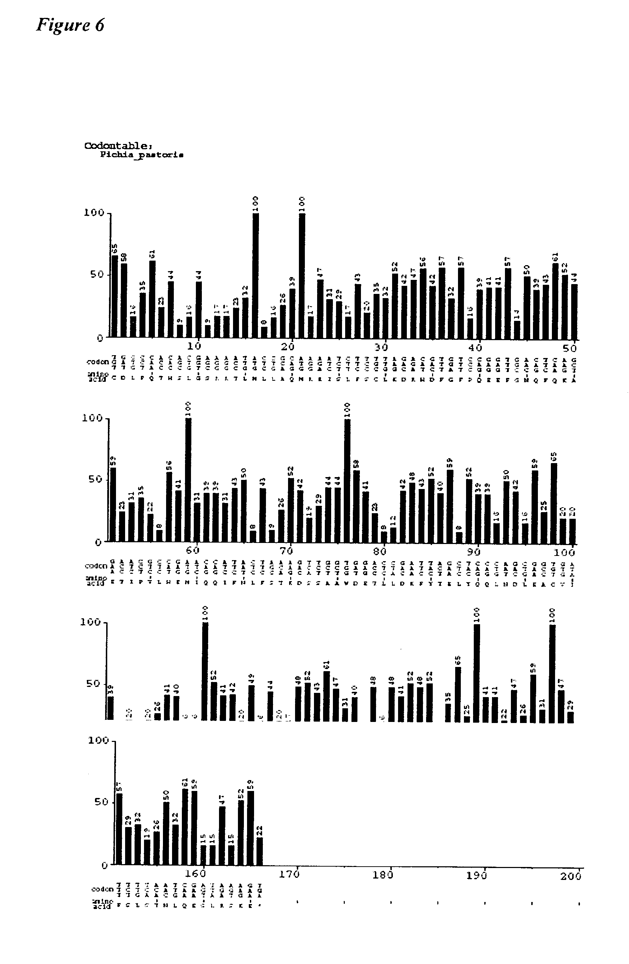

Figure 6: Analysis of the codon usage of the Human IFNα2b encoding cDNA sequence.

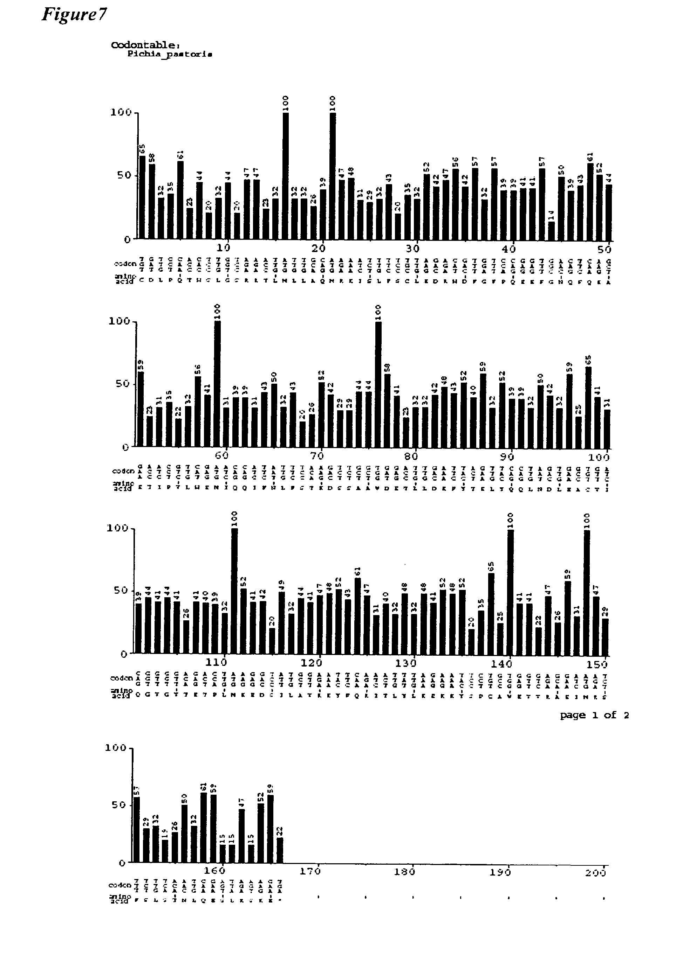

Figure 7: Analysis of the codon usage of the synthetic rHuIFNα2a encoding cDNA sequence.

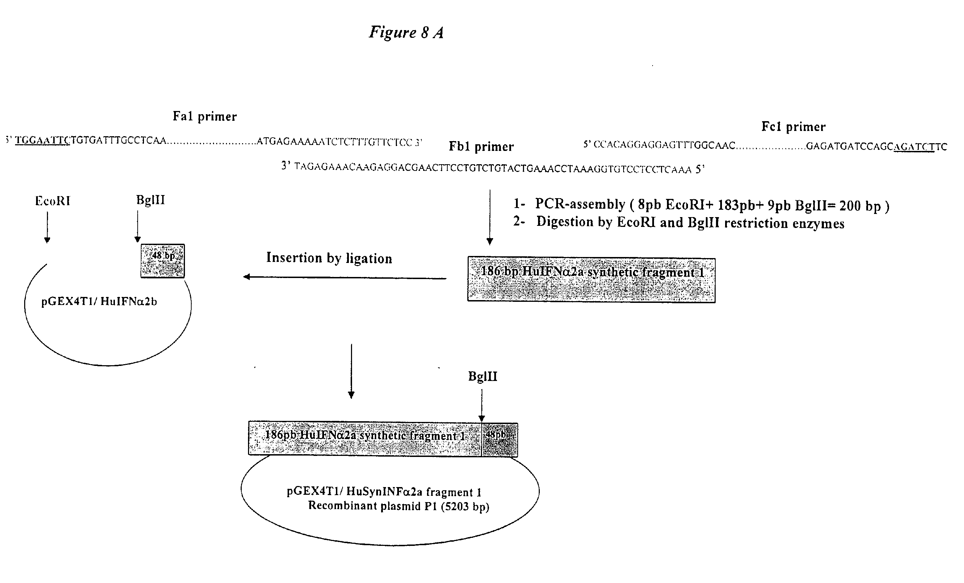

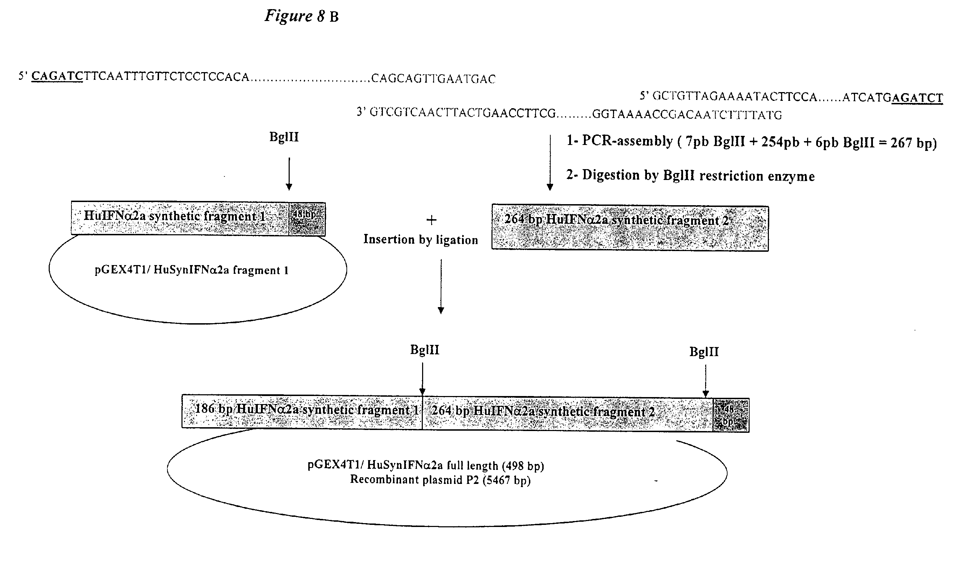

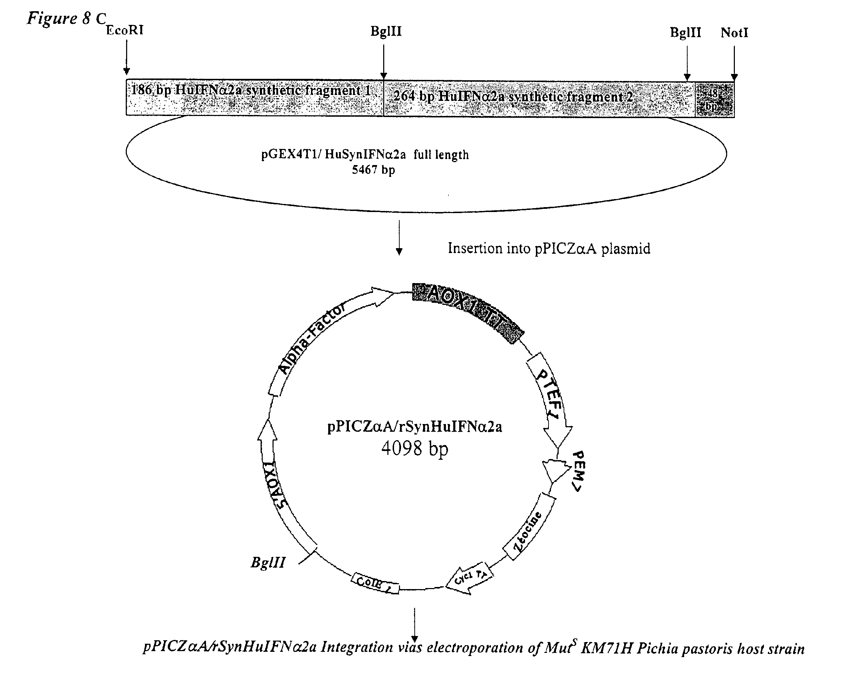

Figure 8: Clone design **Fig. 8A:** Design of the first HuIFNα2a synthetic fragment 1 by PCR-assembly using the synthetic primers: Fa1, Fb1, Fc1. **Fig. 8B:** Design of the second HuIFNα2a synthetic fragment 2 by PCR-assembly using the synthetic primers: Fa2, Fb2 and Fc2. **Fig. 8C:** construction Design of pPICZαA/rSynHuIFNα2a recombinant plasmid.

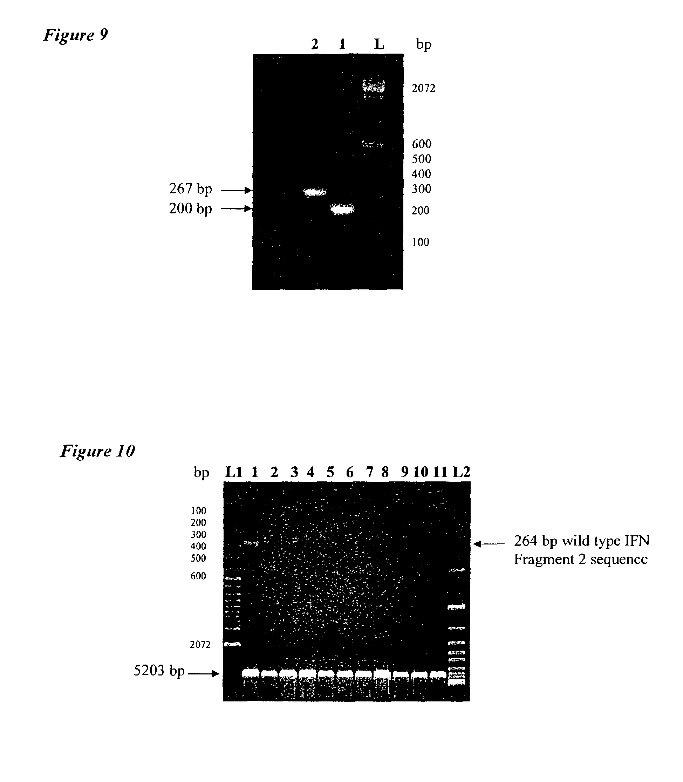

Figure 9: 1% agarose gel Analysis of Purified PCR-Assembly product. Lane 1 represents the 200 bp first synthetic PCR-assembly product using the primers: Fa1, Fb1 and Fc1 **(Figure 8A).** Lane 2 shows the 267 bp second synthetic PCR-assembly product using the primers: Fa2, Fb2, Fc2 **(Figure 8B)**. Lane L, represent the 100 bp DNA Ladder from Invitrogen.

Figure 10: 1% agarose gel Analysis of BglII digested transformed clones Isolation of the P1 recombinant plasmid clone (pGEX4T1 containing the first HuIFNα2a synthetic fragment) was performed by restriction analysis using BglII restriction enzyme as recommended by the manufacturer (Amersham-Biosciences). Lanes L1 and L2 represent respectively the 100bp DNA ladder (Invitrogen) and 1KiloBase DNA marker (Amersham-Biosciences). Lane 1 corresponds to the control BglII digestion product of the recombinant plasmid pGEX4T1/HuIFNα2b. Lanes 2 to 11 correspond to the putative clones containing the 186bp first synthetic IFNα2a sequence.

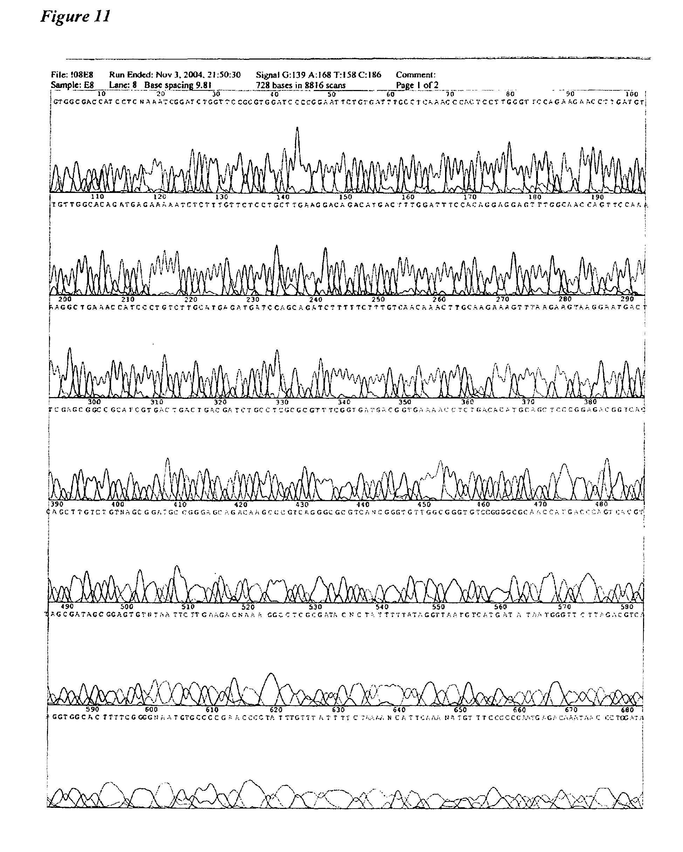

Figure 11: Nucleotide sequence of recombinant plasmid "P1", pGEX4T1 containing the first HuIFNα2a synthetic fragment.

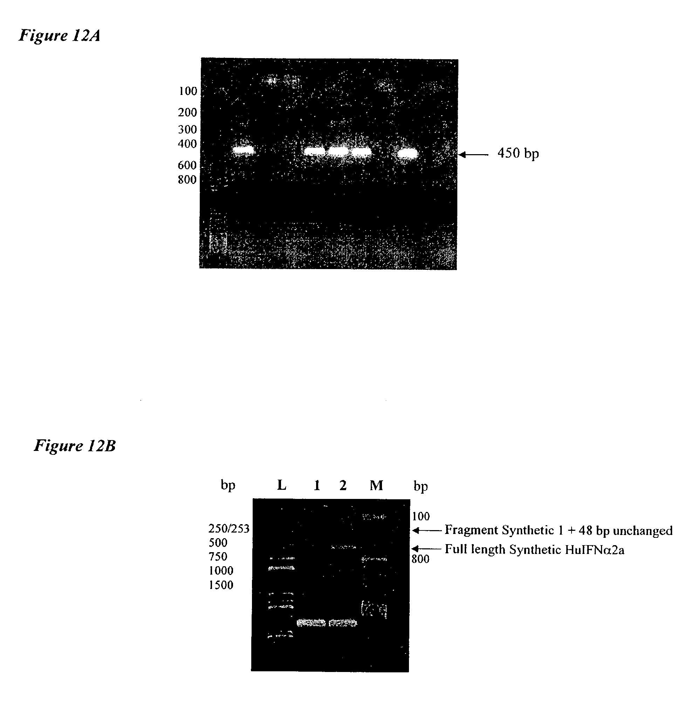

Figure 12: **Fig. 12A:** Isolation of the recombinant plasmid clone pGEX4T1 containing the HuIFNα2a Synthetic fragment 2 inserted into the right sense, was performed by colonies-PCR method using the 5' FfgsI and 3' RfgsII primer pair. Lane 1 for clone 1, 4 for clone 4, 5 for clone 5, 6 for clone 6 and 8 for clone 8, show a 450 bp band corresponding to the amplified PCR product of the 186 bp fragment Synthetic 1 + the 264 bp right sense inserted fragment Synthetic 2. Lane L, represents the 100 base-Pair Ladder from (Amersham-Biosciences). **Fig. 12B** : Isolation of the P2 recombinant plasmid clone was performed by restriction analysis using EcoRI and XhoI restriction enzyme. Lane 2 corresponds to the EcoRI/XhoI digested product from Clone 1 (Figure 12A). The electrophoresed profile shows a 498 bp band corresponding to the 498 pb full length Synthetic Human IFNα2a gene. Lane 1 corresponds to the digested product from P1 cloning vector (pGEX4T1 containing the first HuIFNα2a Synthetic fragment 1+ the native -COOH 48 bp sequence). Lane L represents the 1kb DNA Ladder from Promega, while lane M corresponds to the 100 base-Pair Ladder from (Amersham-Biosciences).

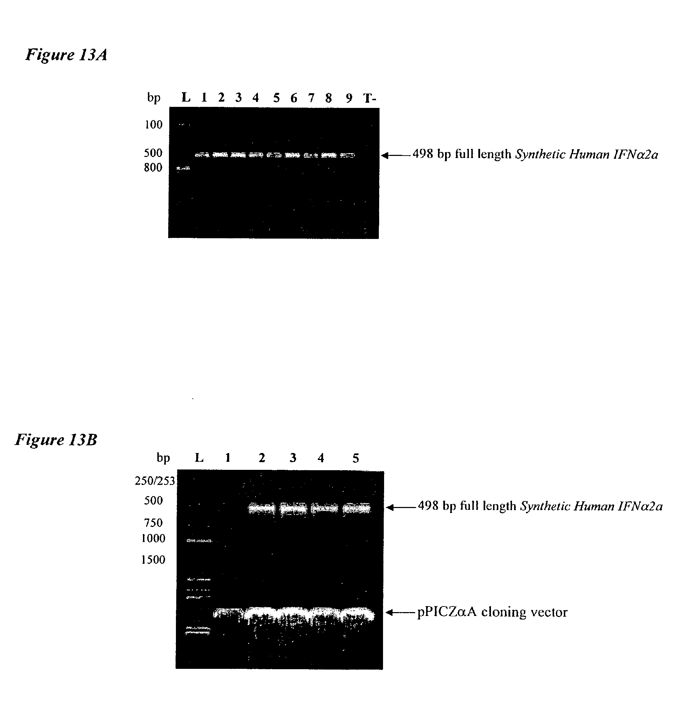

Figure 13: **Fig. 13A:** Isolation of pPICZαA/ Synthetic Human IFNα2a recombinant plasmid containing the cDNA sequence encoding the Synthetic human IFNα2a by "colonies-PCR" based method using the Forward 5' FfgsI and the reverse 3' RIFNα2b primer pair.Clones containing the 498 bp IFNα2a gene (Lane 1, 2, 3, 4, 5, 6, 7, 8, and 9) show a 498 bp PCR-product band, corresponding to the full length Synthetic Human IFNα2a. Lane T- corresponds to the mixed PCR solution. Lane L represents the 100 base-Pair Ladder from Amersham-Biosciences. **Fig. 13B :** Isolation of pPICZαA/Synthetic Human IFNα2a recombinant plasmid containing the cDNA sequence encoding the Synthetic Human IFNα2a by restriction analysis using EcoRI and NotI restriction enzyme. Lane 1 corresponds to the EcoRI/NotI pPICZαA cloning vector digested product. Lanes 2, 3, 4 and 5 represent respectively the clones 2, 3, 4 and 5 containing the 498 bp Synthetic Human IFNα2a. Lane L represents the 1kb DNA Ladder from Promega.

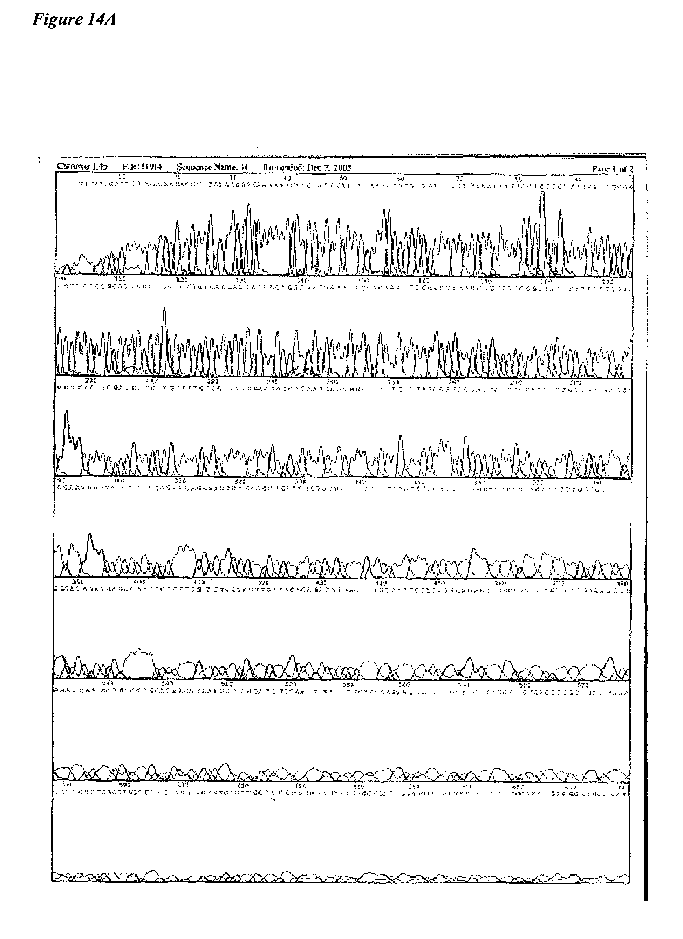

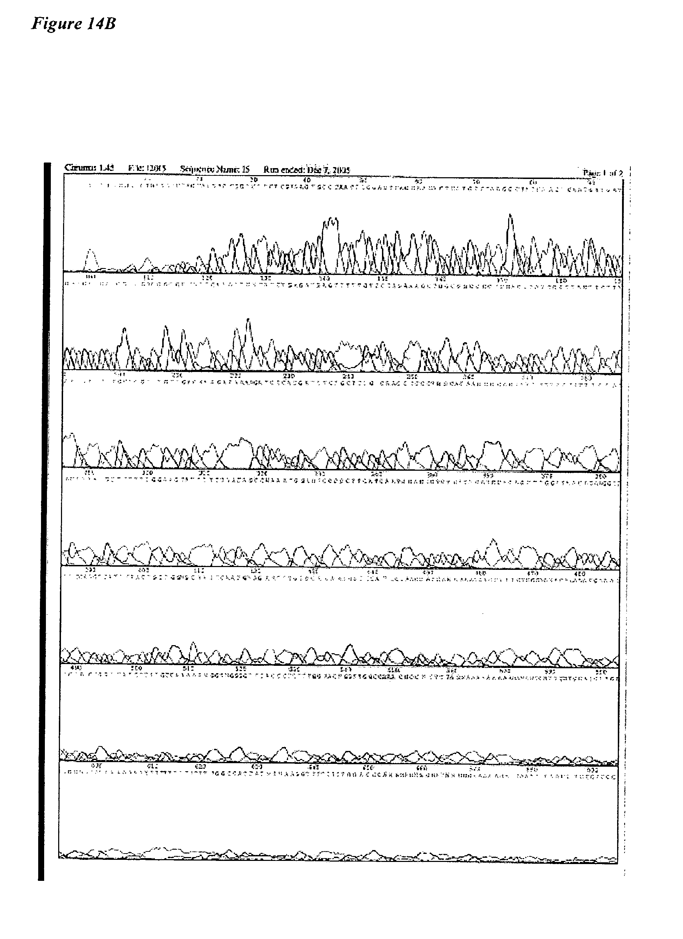

Figure 14: **Fig. 14A:** pPICZαA/ Synthetic Human IFNα2a recombinant plasmid DNA Sequencing Result using the forward Aoxl/Fsequencing primer. **Fig. 14B :** pPICZαA/ Synthetic Human IFNα2a recombinant plasmid DNA Sequencing Result using the reverse Aoxl/R sequencing primer.

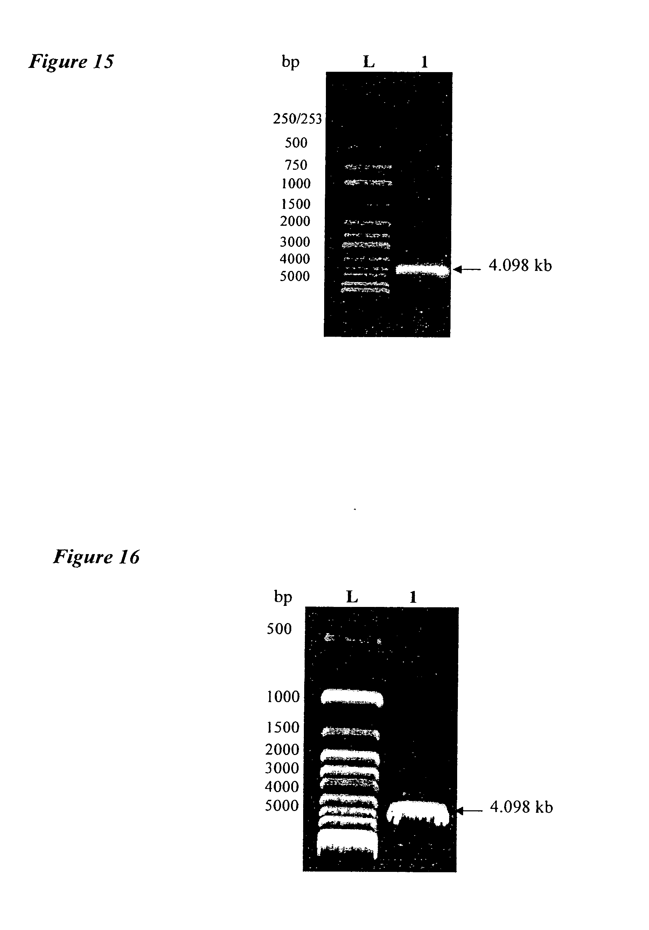

Figure15: The SacI digested product from the recombinant pPICZαA/Synthetic Human IFNα2a. Lane 1 shows the electrophoresis profile on 0.7% agarose gel, TBE 1X of 2 µl phenol/Chloroform/IAA purification product from the digested SacI pPICZαA^{∼}/ Synthetic Human IFNα2a recombinant plasmid. Lane L represents the 1kb DNA Ladder from Promega.

Figure16: The BstXldigested product of the recombinant pPICZαA/rHuIFNα2b. Lane 1 shows the electrophoresis profile on 0.7% agarose gel, TBE 1X of 5 µl phenol/Chloroform/AIA purification product from the digested BstXI pPICZαA^{∼}/rHuIFNα2b recombinant plasmid. Lane L represents the 1KiloBase DNA marker (Amersham-Biosciences).

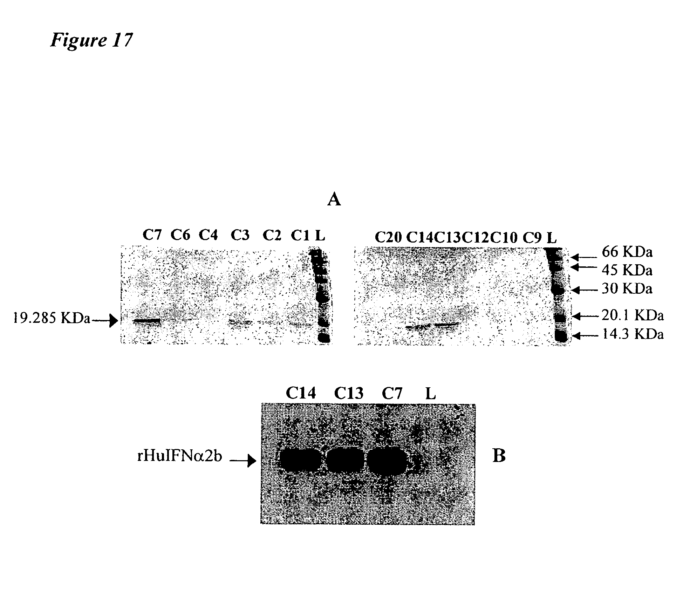

Figure17: **Fig. 17A:** rHuIFNα2b level obtained after 48 h of methanol induction from different selective Zeocin clone's growth (A) Coomassie-stained-SDS-PAGE gel (15%). The arrow indicates the position of rHuIFNa2b; while C1, C2, C3, C4, C6, C7, C9, C10, C12, C13, C14 and C15 stands for the corresponding number of clone. C1, C2, C3, and C12 were only selected on 500µg/ml zeocin YPD plates, clones 4, 6, 10, 20 were only selected on 1000µg/ml zeocin YPD plates, while C7, 13 and 14 were selected on 2000µg/ml zeocin YPD plates. **Fig. 17B:** Immunoblot with Anti- human IFNa polyclonal antibody of the highest level producing clone. Aliquots of 10µl from each recombinant KM71H culture supernatants were electrophoresed on a denaturized conditions with the RPN756 MW markers (lane L for A and B figure).

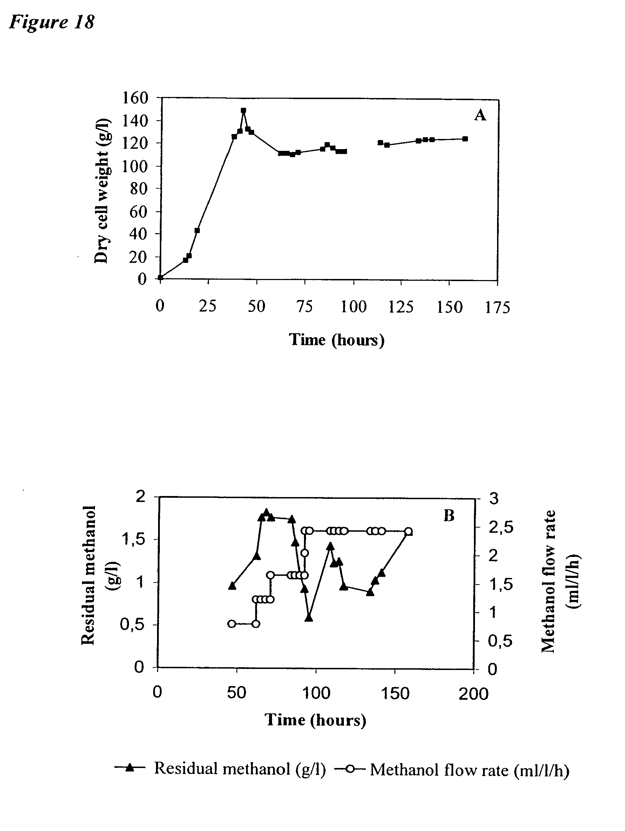

Figure 18: Evolution of biomass **(Fig. 18A)**, residual methanol and methanol flow rate **(Fig. 18B),** through out the culture of the recombinant clone of *Pichia pastoris* in a 5-1 bioreactor producing IFNα2b, on a minimal salt medium.

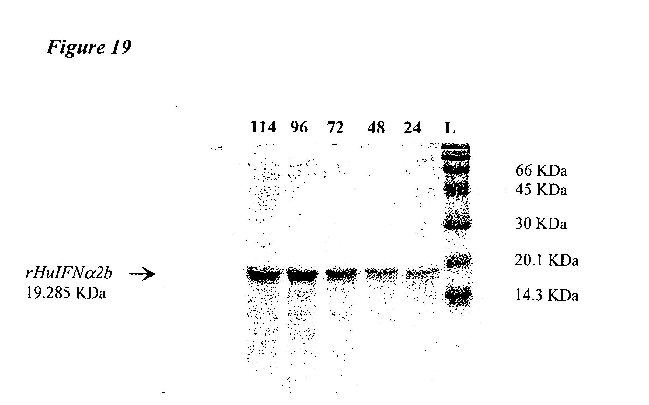

Figure 19: SDS-PAGE 15% analysis of culture supernatant at different induction time of the recombinant clone of *Pichia pastoris* methanol fed batch culture.

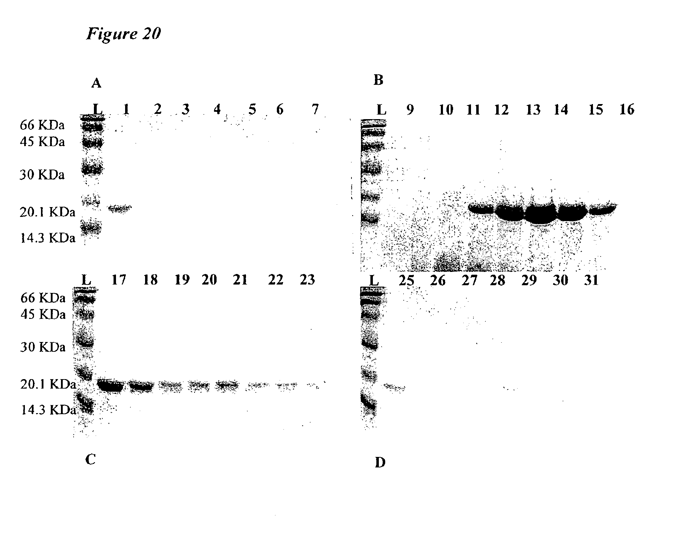

Figure 20: Cation exchange chromatographic purification of rHuIFNα2b on SP-Sepharose column. Coomassie blue stained SDS-PAGE analysis of column fractions. Gel A : Lane 1 shows the crude supernatant, Lane 2 shows the flow through fraction, Lanes 3, 4, 5, 6, 7 correspond to washing fractions. Lane 8 shows the 0.1 M NaCl lactic acid 50 mM 1ml elution fraction. Gels B, C and D show elution fractions with 27 ml step gradient from 0.2 to 1 M NaCl/ lactic acid 50 mM pH 4 (lanes 9 to 32).

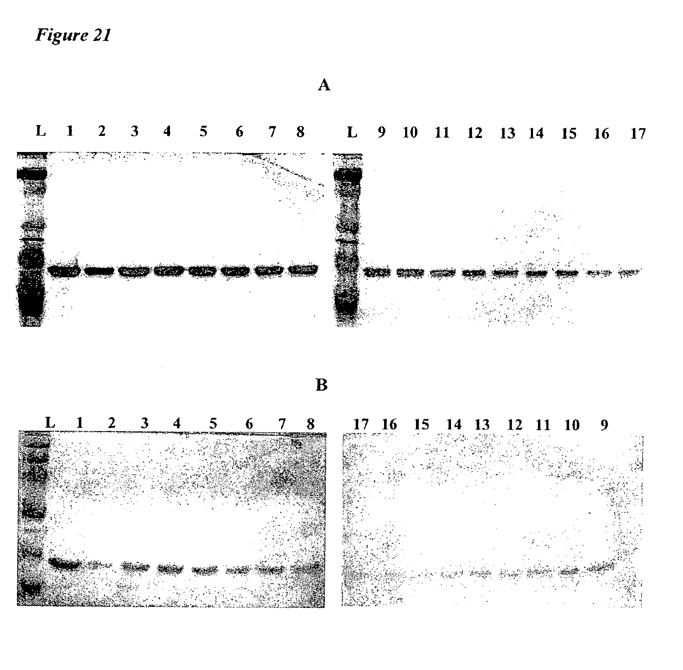

Figure 21: Silver **(A)** and Coomassie blue **(B)** stained SDS-PAGE analysis through size exclusion purification step. The pooled fractions (from fractions 12 to 25) obtained during cation exchange purification on SP sepharose column. Lanes 2 to 17 correspond to the different fractions collected during size exclusion step on Sephacryl S-100 column. Lane L shows RPN756 MW marker.

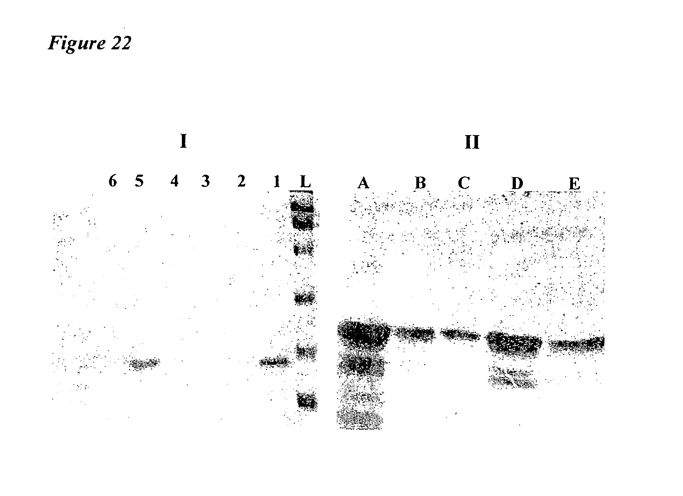

Figure 22: Silver **(I)** and Coomassie Blue **(II)** stained SDS-PAGE analysis through Streamline SP-XL purification step. Lanes 1 and A represent the harvest from HCD culture diluted to OD₆₀₀ to 100. Lanes 2 and B represent the flow through fraction. Lane 3 shows the first wash fraction. Lanes 4 and C, 5 and D, and 6 and E represent the eluted fraction with Lactic Acid 25mM 1M NaCl. Lane L shows RPN756 MW marker.

### EXAMPLES

### Example 1: Construction of Recombinant pPICZαA/rHuIFNα2b expression vector

Human Interferon α2b cDNA was cloned by an RT-PCR approach using mRNA prepared according to standard procedures, isolated from healthy individual leukocytes exposed *in vitro* to the New Castle Disease virus. The cDNA was amplified using the FIFNα2b Forward primer designed to introduce an EcoRI site at the 5' end of the gene (TGGAATTCTGTGATCTGCCTCAAACCCA (SEQ ID NO: 5)) and the RIFNα2b reverse primer designed to contain the NotI site and an TGA STOP codon at the 3' end of the gene (ATTCTGCGGCCGCTCATTCCTTACTTCTTAAACTTTC (SEQ ID NO: 6)). The 498 bp purified RT-PCR product *(**Figure 1**)* was digested with EcoRI and NotI restriction enzymes and inserted into the (3.6 kb) plasmid pPICZαA (Invitrogen, Groningen, the Netherlands) to generate the pPICZαA/rHuIFNα2b expression vector. The *E.coli* Top10F' (*rec*A*⁻*/*endA⁻*) transformed clones were selected on low salt Luria-Bertani (LB) medium with 25µg/ml zeocin. Isolation of pPICZαA/rHuIFNα2b recombinant plasmid containing the cDNA sequence encoding human IFNα2b was performed by colonies-PCR method using the 5' FIFNα2b Forward and 3' RIFNα2b reverse specific primers *(****Figure* 2**), according to procedures described in Current Protocols in Molecular Biology (Ausubel et al.) and by restriction analysis using BglII restriction enzyme as recommended by the manufacturer (Amersham-Biosciences) *(****Figure* 3***)*. Finally, the nucleotide sequence of the positive clones was checked by automated DNA Sequencing Analysis using the "ABI-PRISM ³⁷⁷" DNA sequencer of Perkin-Elmer Applied Biosystem. The Aox1/F ***(Figure 4a)*** and Aoxl/R ***(Figure 4b)*** from the "Easy select *Pichia* protein expression kit" (Invitrogen, Groningen, the Netherlands), were used as the sequencing primers.

### 1.1 pPICZαA cloning vector features

pPICZαA cloning vector has been designed as *Escherichia coli*/*P. pastoris* shuttle vector, containing:
- An origin of replication for plasmid propagation and maintenance in *E.coli* strain line, the ColE1 origin (pUC-derived): bases 2866-3539.
- For both *P. pastoris* and *E.coli,* the selectable marker gene, is the *Sh ble* gene from *Streptoalloteichus hindustanus:* bases 2163-2537 that confers resistance to bleomycin-related drug zeocin (Cregg et al., 1985; Cregg et al., 1989a; Higgins et al., 1998) With a transcription elongation factor I gene promoter (TEF I promoter/ GenBank accession number D12478, D01130/ bases 1683-2094) from *Saccharomyces cerevisiae* that drives expression of *Sh ble* gene in *P.pastoris* strain, and the *E.coli* constitutive promoter droved *Sh ble* gene expression: the EM7 (synthetic prokaryotic promoter): bases 2095-2162.
- An 1202 bp fragment expression cassette from AOX1 gene composed of the 5' AOX1 promoter (bases: 1-940) that allows methanol-inducible, high level expression in *P.pastoris,* and a second short sequence of 260 bp required for transcription termination *TT*: bases 1341-1601 (Koutz et al., 1989) that permits efficient 3' RNA processing, including polyadenylation: base 1520, for increased mRNA stability.
- Between the promoter and the terminator sequence there is the multiple cloning sites (MCS), bases 1208-1274, containing the 10 unique restriction enzyme sites for insertion of foreign sequences.
- In the pPICZαA AOX1 gene, the alcohol oxidase open frame (ORF) is preceded by an unusually long 5' untranslated region (UTR) of 823 bp: bases 1-823.
- The 5' end of AOX1 mRNA: base 824 and the 3' end of mRNA: base 1514.
- The *Saccharomyces cerevisiae* α-factor secretion signal (bases 941-1207), allows the secretion of most proteins from *P.pastoris* host strain.
- The initiation ATG (bases 941-943) in the α-factor signal sequence in pPIZCαA corresponds to the native initiation ATG of the AOX1 gene.
- The priming site of sequencing primers used to check the integrity of the plasmid construction was respectively: the 5' AOX1 priming site: bases 855-875 and the 3' AOX1 priming site: bases 1423-1443.
- For expression of C-terminal fusion tagged proteins, pPICZαA contain both C-terminal *myc* epitop tag (Glu-Gln-Lys-Leu-Ile-Ser-Glu-Glu-Asp-Leu-Asn (SEQ ID NO: 7)): bases 1275-1307, that permits detection of fusion protein by anti-*myc* Antibody; and the C-tenninal polyhistidine tag: bases 1320-1340 that encode 6 histidine that form a metal binding site for affinity purification of fusion protein.
- The SacI **(base: 209),** PmeI **(base: 414),** and BstX1(**base: 707)** are the unique restriction enzymes that we can use to permit linearization of the recombinant vector at the 5' AOX1 locus for efficient integration into *P.pastoris* genome.

### 1.2 pPICZαA/rHuIFNα2b recombiant plasmid features

The recombinant expression plasmid pPICZαA/rHuIFNα2b (Figure 5) was constructed using the pPICZαA cloning vector as described in Example 1.1. The gene coding for recombinant Human Interferon α2b (rHuIFNα2b) was cloned in frame and downstream of the α-factor signal sequence at the 5' in frame EcoRI restriction site and the 3' NotI restriction site. To express rHuIFNα2b without any C-terminal peptide (C-terminal *myc* epitop tag and C-terminal polyhistidine tag), we introduced at the 3' end of gene a TGA STOP codon; resulting in a native C-terminal HuIFNα2b secreted protein ***(Figure 5).*** The TGA stop codon was introduced in the RIFNα2b reverse primer that was used to amplify the human alpha interferon cDNA. This primer was designed so as to introduce the TGA stop codon in frame with cDNA coding sequence.

### 1.3. Bacterial media composition:

To have active zeocin, salt concentration in the medium must remain low (< 90 mM) and the pH must be equal to 7.5. Low salt LB medium contains 1 % Tryptone, 0.5 % NaCl and 0.5 % Yeast extract at pH 7.5. Zeocin was added to a final concentration of 25 µg/ml.

### Example 2: Construction of Recombinant pPICZαA/rHuIFNα2a expression cassette with optimized codon usage

The concept that organisms display a non-random pattern of synonymous codon usage was established by Grantham et al. (1980) and has been confirmed by the explosion of sequence data available from recent genome sequencing projects (Nakamura et al., 1999). Moreover, all organisms investigated to date show some general bias towards a subset of the 61 possible sense codons (Nakamura et al., 1999; Li et al., 1991). While several hypotheses have been reported to explain the origin of this bias, a model involving selection for translational efficiency has been well supported for prokaryotes, unicellular eukaryotes, and to some extent, for insects (Bagnoli et al., 1995; Bulmer, 1987; Powell and Moriyama, 1997). Significant experimental work has been done to examine the effect of varying codon bias in heterologous expression systems, with the assumption that disparate patterns of codon bias between the cloned gene and the expression host will have significant impact on the level of recombinant protein produced. The optimization of coding regions towards the codon bias of the host cell has led to an average 10 to 50 fold increase in heterologous protein production in several host cells expression (Andre et al., 1998; Apeler et al., 1997; Brocca et al., 1998; Nagata et al., 1999; Ushijima et al., 1998; Zolotukhin et al., 1996).

The inventors have decided to study whether codon optimization of the Human Interferon α 2a cDNA towards the bias of *P. pastoris* could have a positive impact on expression levels. Hence, they have synthesized an artificial gene, according to the codon usage bias of *P. pastoris* as reported by Sinclair et al. (2002), as decribed below.

### 2.1 Design of a Pichia pastoris codon-optimized human interferon α2a cDNA and construction of the expression cassette

Leukocytes were isolated from a healthy individual and exposed in vitro to the New Castle Disease virus. RNA was prepared from these infected cells and used to copy the human IFNα2b mRNA by RT/PCR. The IFNα2b cDNA was cloned into the PICZαA plasmid and its nucleotide sequence determined and analysed by using the nearest neighbor analysis "graphical codon usage analyzer" software available through the WEB at **http://www.geneart.degcua.schoedl.de/** or from ExPASy Proteomics tools at www.Swissprot.org **(Figure 6)**.

The inventors have decided to change both the R (AGA) at position 23 to L (AAA) and the rarest codons according to the codon usage bias of *P. pastoris* as reported by Sinclair et al. (2002). Once the codons to be changed were chosen, the novel Human IFNα2a cDNA sequence was analysed "graphical codon usage analyzer" software as described above. The result shown in **Figure 7,** confirms that the rHuIFNα2a synthetic sequence contain only the codon usage bias of *Pichia pastoris* organism.

The codon analysis of the synthetic Human IFNα2a sequence **(Figure 7),** was used to design the first HuIFNα2a synthetic fragment 1 obtained by PCR-assembly method using the synthetic primers: Fa1 (SEQ ID NO: 8), Fb1 (SEQ ID NO: 9) and Fc1 (SEQ ID NO: 10) and the two external primers: the 5' forward FfgsI (TGGAATTCTGTGATTTGCCTCA (SEQ ID NO: 11)) and the 3' reverse RfgsI primer (GAAGATCTGCTGGATCATCTC (SEQ ID NO: 12)). While, primers: Fa2 (SEQ ID NO: 13), Fb2 (SEQ ID NO: 14), Fc2 (SEQ ID NO: 15) were used to generate the second synthetic Human IFNα2a cDNA fragment 2, using the two external primers: the 5' forward FfgsII (TGAAGATCTTCAATTTGTTCTCCAC (SEQ ID NO: 16)) and the 3' reverse RfgsII primer (TGAAGATCTCATGATTTCTGCTCTGA (SEQ ID NO: 17)). Details steps of the cloning strategy are depicted in **Figure 8A, B and C**.

Gene synthesis and fragment assembly were based on the PCR method described in **Figure 8A and 8B,** using *Taq DNA polymerase* from Amersham-Biosciences.

The purified 200 bp DNA fragment corresponding to the first HuIFNα2a synthetic fragment PCR-assembly product **(Figure 9, lane 1)** was cloned into the recombinant plasmid pGX4T1/rHuIFNα2b previously restricted at the 5' *EcoR*I and the 3' *Bgl*II restriction sites, generating a plasmid containing only the 48 bp corresponding to the 16 amino acids HuIFNα2a COOH terminus. According to the codon bias usage in *P.pastoris,* the 16-C-terminal amino acids of the HuIFNα2a does not contain any rare codon.

Isolation of the P1 **(Figure 8A)** recombinant plasmid clone (pGEX4T1 containing the first HuIFNα2a synthetic fragment) was performed by restriction analysis using *Bgl*II restriction enzyme **(Figure 10)** as recommended by the manufacturer (Amersham Biosciences). Finally, the sequences of the positive clones were checked by DNA Sequence Analysis using the "ABI-PRISM ³⁷⁷" DNA sequencer of Perkin-Elmer Applied Biosystem. The 5'pGEX primer was used as the sequencing primer **(Figure 11)**.

The second 267bp HuIFNα2a synthetic fragment PCR-assembly product ***(Figure 9, lane* 2)** was introduced into the BglII digested P1 recombinant plasmid.

Isolation of the P2 ***(Figure 8B)*** recombinant plasmid clone pGEX4T1 containing the HuIFNα2a Synthetic fragment 2 inserted into the right sense, was performed by colonies-PCR method using the 5' FfgsI Forward (SEQ ID NO: 10) and the 3' RfgsII reverse specific primers (SEQ ID NO: 16) ***(Figure 12A)*** and by restriction analysis using EcoRI+ XhoI restriction enzymes as recommended by the manufacturer (Amersham-Biosciences)) ***(Figure 12B)**.*

The full length synthetic HuIFNα2a was digested with *EcoR*I and *Not*I restriction enzymes and inserted into the pPICZαA plasmid to generate the pPICZαA/rSynHuIFNα2a expression vector **(Figure 8C).**

The *E.coli* Top10F' *(rec*A*⁻*/*end*A*⁻)* transformed clones were selected on low salt LB medium with 25 µg/ml zeocin. Isolation of pPICZαA/ Synthetic HuIFNα2a recombinant plasmid containing the cDNA sequence encoding synthetic human IFNα2a was performed by colonies-PCR method using the 5' FfgsI Forward (SEQ ID NO: 11) and the 3' RIFNα2b reverse specific primers (SEQ ID NO: 6) ***(Figure 13A),*** according to procedures described in Current Protocols in Molecular Biology (Ausubel et al.) and by restriction analysis using *EcoR*I and *Not*I restriction enzymes as recommended by the manufacturer (Amersham-Biosciences) ***(Figure 13B)***. Finally, the nucleotide sequence of the positive clones was checked by automated DNA Sequencing Analysis using the "ABI-PRISM ³⁷⁷" DNA sequencer of Perkin-Elmer Applied Biosystem. The Aoxl/F ***(Figure 14A)*** and Aoxl/R ***(Figure 14B)*** from the "Easy select *Pichia* protein expression kit" (Invitrogen, Groningen, the Netherlands), were used as the sequencing primers.

### 2.2 pPICZαA/rSynHuIFNα2a Integration via electroporation of Mut^{s} KM71H Pichia pastoris host strain

The recombinant pPICZαA/rSynHuIFNα2a *P.pastoris* expression vector was propagated in *E.coli* strain Top10F' *(rec*A⁻/*endA⁻)* in presence of 25 µg/ml zeocin. Plasmid DNA was isolated using Qiagen plasmid miniprep kit from transformed *E.coli* clones. It was digested according to manufacturer's instruction, by *Sac*I restriction enzyme that does not cut within rSynHuIFNα2a cloned gene (Amersham-Biosciences). The digested DNA was purified by phenol/chloroform/IAA purification method following standard protocols ***(Figure 15).*** Ten µg of linearized recombinant plasmid DNA was used to transform KM71H (aox1::ARG4) *P.pastoris* strain via electroporation method (Bio-Rad GenePulser, 1500 charging voltage (V), 25 µF capacitance, and 200 resistance), as described in the *EasySelect^{™} Pichia protein expression manual* (Invitrogen Catalogue No. K1740-01). After 3 days of incubation at 30°C on YPDS solid medium containing zeocin at 100 µg/ml, 20 KM71H Mut*^{S}* transformant clones were retained for further study. Gene insertion occurred at the *aox1::ARG4* (KM71H) loci from a single crossover event between the loci of the two AOX1 regions on the pPIZαA vector: the AOX1 promoter or the AOX1 transcription terminator (TT). This results in insertion of one or more copies of the recombinant plasmid (pPICZαA/rSynHuIFNα2a) upstream or downstream of the *aox1::ARG4* gene.

### Example 3. Transformation of Pichia pastoris Mut^{s} KM71H (aoxl::ARG4) host strain

### 3.1 Pichia.pastoris Mut^{S} KU71H phenotype

Strains with AOX mutations are sometimes better producers of foreign proteins than wild type strains (Tschopp et al., 1996; Chiruvoulu et al., 1997). Additionally, these strains do not require the large amounts of methanol routinely used for large-scale fermentation of Mut⁺ strains. *Pichia pastoris* KM71H (His4 aox1::ARG4) is a strain in which the codons 16 through 227 of AOX1 have been replaced by the *S. cerevisiae* wild type ARG4, creating a disturbed AOX1 gene (Cregg and Madden, 1987a). Since the strain must rely on the weaker AOX2 for methanol metabolism, AOX1-disturbed clone shows a slower growth phenotype (Mut^{s}) on minimal methanol medium as compared with Mut⁺ strain (Sreekrishna et al., 1997). However, since the AOX1 locus was not completely deleted, these Mut^{s} strains retain the ability to induce expression at high levels from the AOX1 promoter (Chiruvoulu et al., 1997).

### 3.2 pPICZαA/rHuIFNα2b Integration via electroporation of Mut^{s} KM71H Pichia pastoris host strain

The recombinant pPICZαA/rHuIFNα2b *P.pastoris* expression vector was propagated in *E.coli* strain Top10F' (*rec*A⁻/*endA⁻*) in presence of 25 µg/ml zeocin. Plasmid DNA was isolated using Qiagen plasmid miniprep kit from transformed *E.coli* clones. It was digested by *BstX*1 restriction enzyme that does not cut within the rHuIFNα2b cloned gene (Amersham-Biosciences). The digested DNA was purified by phenol/chloroform/AIA purification method following standard protocols ***(Figure 16)**.* Ten µg of linearized recombinant plasmid DNA were used to transform KM71H (aox1::ARG4) *P.pastoris* strain via electroporation method (Bio-Rad GenePulser, 1500 charging voltage (V), 25 µF capacitance, and 200 ohms resistance), as describe in the *EasySelect^{™} Pichia protein expression manual* (Invitrogen, Catalogue N°. K1740-01). After 3 days of incubation at 30°C on YPDS solid medium containing zeocin at 100µg/ml, 20 KM71 H Mut^{s} transformant clones were retained for further study. Gene insertion occurred at the *aox1::ARG4* (KM71 H) loci from a single crossover event between the loci of the two AOX1 regions on the pPICZαA vector: the AOX1 promoter or the AOX1 transcription terminator (TT). This results in insertion of one or more copies of the recombinant plasmid (pPICZαA/rHuIFNα2b) upstream or downstream of the *aox1::ARG4* gene.

### Example 4: Isolation of Multi-Copy expression clones

Numerous examples reporting the effect of expression cassette copy number on protein production in *P.pastoris* were reported (Sreekrishna et al., 1997; Clare et al., 1991a; Sreekrishna et al., 1989; Romanos et al., 1991; Clare et al., 1991b; Sreekrichna 1993). However, a strain that contains a single copy of the expression cassette can be sufficient for optimal production (Cregg et al., 1985; Cregg et al., 1987b, Sreekrishna et al., 1990; Sreekrishna et al., 1993). Additionally, in some cases, an increase in copy number has a negative effect on the production level (Thill et al., 1990). Thus, the effect of gene copy number is unpredictable. A solution to this problem is to evaluate the production level of different clones as a function of gene dosage, by employing clones selection based on increased level of resistance to an antibiotic marker (Higgins et al., 1998; Clare et al 1991 a; Scorer et al., 1994). Thus, to isolate the multi-copy expression clones, a quick and directed way based on the selective clones' growth on increasing concentration of zeocin (Higgins et al., 1998) was carried out. Furthermore, strains transformed with expression cassettes containing the zeocin can be selected directly by resistance to the drug marker by plating the putative recombinants clones on YPDS plates containing different concentrations of zeocin: 500, 1000 and 2000 µg/ml. Additionally, populations of transformants can be screened for multicopy expression cassettes strains, simply by plating on increased concentration of zeocin in the selection plates. In the present case, the inventors found a correlation with the highest zeocin resistance clones and the highest expression level clones ***(Figure 17)**.*

Three clones : 7, 13 and 14 were able to growth on 2000 µg/ml zeocin plate. The highest level rHuIFNα2b producing *P.pastoris* clones (clones 7, 13, and 14) were identified by a comparative analysis with 12 different selective zeocin clones' growth (clones : 1, 2, 3, 4, 6, 7, 9, 10, 12, 13, 14, and 20). Visual comparison of band intensity of SDS-PAGE Coomassie Brillant Blue stained ***(Figure 17A)*** and western blot ***(Figure 17B)*** of the culture supernatants, was carried out. One of the three highest producing rHuIFNα2b clones (Clone 7) was chosen for high cell density culture.

### Example 5: Synthetic primers, cDNA and Amino acid sequences of the two engineered genes of Pichia pastoris clones producing human IFNα

### Synthetic rHuIFNα2a (AAA/23) cDNA sequence

5'TGTGATTTGCCTCAAACCCACTCCTTGGGTTCCAGAAGAACCTTGATGTTG
TTGGCACAGATGAGAAAAATCTCTTTGTTCTCCTGCTTGAAGGACAGACATGACTTTGGATTTCC
ACAGGAGGAGTTTGGCAACCAGTTCCAAAAGGCTGAAACCATCCCTGTCTTGCATGAGATGATC
CAGCAGATCTTCAATTTGTTCTCCACAAAGGACTCTTCTGCTGCTTGGGATGAGACCTTGTTGG
ACAAATTCTACACTGAATTGTACCAGCAGTTGAATGACTTGGAAGCCTGTGTTATCCAGGGTGT
TGGTGTTACAGAGACTCCATTGATGAAGGAGGACTCCATTTTGGCTGTTAGAAAATACTTCCAA
AGAATCACTTTGTATTTGAAAGAGAAGAAATACTCCCCTTGTGCCTGGGAGGTTGTCAGAGCAG
AAATCATGAGATCTTTTTCTTTGTCAACAAACTTGCAAGAAAGTTTAAGAAGTAAGGAATGA 3'
(SEQ ID NO: 1)

### Synthetic rHuIFNα2a (K/23) Amino Acid sequence NH2-

CDLPQTHSLGSRRTLMLLAQMRKISLFSCLKDRHDFGFPQEEFGNQFQKAETIPVLHEMIQQIFNLFS
TKDSSAAWDETLLDKFYTELYQQLNDLEACVIQGVGVTETPLMKEDSILAVRKYFQRITLYLKEKK
YSPCAWEVVRAEIMRSFSLSTNLQESLRSKE- COOH (SEQ ID NO: 2)

### rHuIFNα2b (AGA/23) cDNA inframe Sequence

5'TGTGATCTGCCTCAAACCCACAGCCTGGGTAGCAGGAGGACCTTGATGCT
CCTGGCACAGATGAGGAGAATCTCTCTTTTCTCCTGCTTGAAGGACAGACATGACTTTGGATTT
CCCCAGGAGGAGTTTGGCAACCAGTTCCAAAAGGCTGAAACCATCCCTGTCCTCCATGAGATGA
TCCAGCAGATCTTCAATCTCTTCAGCACAAAGGACTCATCTGCTGCTTGGGATGAGACCCTCCT
AGACAAATTCTACACTGAACTCTACCAGCAGCTGAATGACCTGGAAGCCTGTGTGATACAGGG
GGTGGGGGTGACAGAGACTCCCCTGATGAAGGAGGACTCCATTCTGGCTGTGAGGAAATACTT
CCAAAGAATCACTCTCTATCTGAAAGAGAAGAAATACAGCCCTTGTGCCTGGGAGGTTGTCAGA
GCAGAAATCATGAGATCTTTTTCTTTGTCAACAAACTTGCAAGAAAGTTTAAGAAGTAAGGAAT
GA 3' (SEQ ID NO: 3)

### HUMAN IFN ALPHA 2b (R/23) AMINO ACID SEQUENCE NH2-



- COOH (SEQ ID NO: 4)

### Fa1

5'-
TGGAATTCTGTGATTTGCCTCAAACCCACTCCTTGGGTTCCAGAAGAACCTTGATGTTGTTGGC
ACAGATGAGAAAAATCTCTTTGTTCTCC-3'     (SEQ ID NO: 8)

### Fb1

5'-AAACTCCTCCTGTGGAAATCCAAAGTCATGTCTGTCCTTCAAGCAGGAGAACAAAGAGAT-3' **(SEQ ID NO: 9)**

### Fc1

5'-
CCACAGGAGGAGTTTGGCAACCAGTTCCAAAAGGCTGAAACCATCCCTGTCTTGCATGAGATGA
TCCAGCAGATCTTC-3' (SEQ ID NO: 10)

### FfgsI

5'-TGGAATTCTGTGATTTGCCTCA-3' **(SEQ ID NO: 11)**

### RfgsI

5'-GAAGATCTGCTGGATCATCTC-3' **(SEQ ID NO: 12)**

### Fa2

5'-
CAGATCTTCAATTTGTTCTCCTCCACAAAGGACTCTTCTGCTGCTTGGGATGAGACCTTGTTGGA
CAAATTCTACACTGAATTGTACCAGCAGTTGAATGAC-3' (SEQ ID NO: 13)

### Fb2

5'-
GTATTTCTAACAGCCAAAATGGAGTCCTCCTTCATCAATGGAGTCTCTGTAACACCAACACCCT
GGATAACACAGGCTTCCAAGTCATTCAACTGCTG-3' (SEQ ID NO: 14)

### Fc2

5'-
GCTGTTAGAAAATACTTCCAAAGAATCACTTTGTATTTGAAAGAGAAGAAATACTCCCCTTGTG
CCTGGGAGGTTGTCAGAGCAGAAATCATGAGATCT-3' (SEQ ID NO: 15)

### FfgsII

5'-TGA**AGATCT**TCAATTTGTTCTCCAC-3' **(SEQ ID NO: 16)**

### RfgsII

5'-TGAA**GATCT**CATGATTTCTGCTCTGA-3' **(SEQ ID NO: 17)**

### Example 6: Shake-flask production of secreted rHuIFNα2b

The Expression of the native C-terminal HuIFNα2b secreted protein is controlled by the methanol-regulated promoter. The *Saccharomyces cerevisiae* α-factor secretion signal drives the rHuIFNα2b expression to the extracellular domain. The α-factor secretion signal is a prepro peptide consisting of a 19-amino acids signal (pre) sequence followed by a 70- amino acids peptide (pro) sequence containing three consensus N-linked glycosylation sites and a 7- amino acids sequence Kex2 endopeptidase processing site (Kugan and Herskwitz 1982). The processing of the α-factor secretion signal involves three steps. The firs step consists in the removal of the pre signal by the signal peptidase in the endoplasmic reticulum. The second step, includes the Kex2 endopeptidase cleavage between Arg-Lys of the pro leader sequence, followed by a third step in which Ste13 protease cleaves after the 2 Glu-Ala repeats residues pro-sequence (Brake et al., 1984).

A single colony of pPICZαA/rHuIFNα2b *Mut^{S} KM71H Pichia Pastoris* transformants was used to inoculate 50ml of BMGY in a 500ml flask and incubated at 30°C and 250 rpm for 24h. The cells were pelleted by centrifugation at 3000 g for 10 min, resuspended in 5ml BMMY medium containing 0.5 % methanol and incubated at 30°C and 250 rpm. During induction, methanol was added every 24h to a final concentration of 1%. After 2 days (48 h) of induction, the cells were harvested by centrifugation at 10000 g for 20 min at 4°C; the supernatant was collected and stored at -20°C for protein expression analysis.

### 6.1. Media composition for the Shake-flask production

Yeast media composition: Yeast extract-peptone-dextrose medium (YPD) contained 2% peptone, 1% yeast extract and 2% dextrose, whereas YPDS was supplemented with 1 M sorbitol. Zeocin was added to a final concentration of 100µg/ml. The buffered minimal glycerol-complex-medium (BMGY) was prepared with 2% peptone, 1% yeast extract, 1% glycerol, 1.34% yeast nitrogen base (YNB) with ammonium sulphate but without amino acids, and 4x10⁻⁵ biotin in 100 mM potassium phosphate buffer at pH 6.0. The buffered minimal methanol-complex-medium (BMMY) has the same composition as BMGY, except that 1% methanol is used instead of glycerol.

### 6.2. Secreted rHuIFNα2b protein expression analysis

To analyse the extracellular expression of rHuIFNα2b recombinant protein in *Mut^{S} KM71H Pichia pastoris* host strain, the supernatants were analysed using SDS-PAGE. Electrophoresis was performed using 15% SDS-polyacrylamide gel stained with Coomassie Brillant Blue as described by Laemilli.

The recombinant fusion protein is specifically detected by Western blot-ECL Assays (Amersham Biosciences) performed according to the manufacturer's instructions using 1:400 dilution of Anti- human IFNa polyclonal antibody (Endogen), followed by the Anti Goat/sheep IgG peroxidase conjugated monoclonal antibody (Sigma) used as the second antibody.

### Example 7: High cell density culture

The recombinant clone of *P. pastoris* isolated from 100 µg zeocin/ml-YPD plate, was grown in 10 ml BMGY medium in a 125 ml baffled flask for 12h at 30°C and 250 rpm.

This culture was used to inoculate 200 ml of BMGY medium in a 2-liter baffled flask for 12 h at 30°C and 250 rpm.

High cell density cultures were carried out in a 5-liter bioreactor, equipped with a process control system of data.

The batch culture was carried out with an initial culture volume of 2 litres inoculated with the 200 ml preculture, the culture was carried out at the following conditions: temperature = 30°C, stirrer speed = 800 rpm, pH maintained at 5 by addition of 25% ammonia, dissolved oxygen was set at 40% of air saturation by stirrer cascading and enrichment of the inlet air with pure oxygen when required.

The fed batch culture on glycerol was started once the optical density at 600 nm (OD) reached 80. After a 24 hours of a fed batch culture on glycerol at a rate equal to 15 ml/l/h, the cell dry weight reached at the end of this phase, was equal to 110 - 120 g/l.

The cells were then harvested from the fermentor centrifuged at 3500 rpm (2000g), 4°C and 15 min; the cells were then resuspended in fresh medium and returned back under aseptic conditions, to the bioreactor containing 2.8 liters of synthetic medium that was supplemented with EDTA at 10 mM (final concentration), which avoids the proteolysis of the protein of interest. A fed batch culture on methanol was then started, the methanol feeding rate was modulated to keep its residual level under 0.6% (v/v). Residual methanol was measured by gas chromatography. The induction phase lasts for 5 days. The yield achieved was equal to 500 -600 mg/l.

The composition of the media used for bioreactor culture were as follows:

Batch medium: glycerol (40 g/l), K₂SO₄ (18.2 g/l), MgSO₄ (7.28 g/l), KOH (4.13 g/l), CaSO₄.2H₂O (0.93 g/l) and 85% orthophosphoric acid (26,7 ml/l) dissolved in deionised water and sterilized in the bioreactor. 5 ml/l basal salts of fermentation PTM1 and 2 ml/l of 500X biotin were added to the medium after sterilization, culture medium pH was adjusted to 5 by addition of 25% ammonia (w/v).

The PTM1 solution contains: CuSO₄.5H₂O (6 g l⁻¹), NaI (0.08 g l⁻¹), MnSO₄.H₂O (3 g l⁻¹), Na₂MoO₄.2H₂O (0.2 g l⁻¹), H₃BO₃ (0.02 g l⁻¹), CoCl₂ (0.5 g l⁻¹), ZnCl₂ (20 g l⁻¹), FeSO₄.7H₂O (65 g l⁻¹), biotin (0.2 g l⁻¹) and H₂SO₄ 98% (5 ml l⁻¹).

Fed-batch medium contains glycerol (450 g/1), PTM1 (8 ml/l) and 500X biotin (5 ml/l). Methanol fed-batch solution contains methanol at 100% (973 ml/l), 500X biotin (5 ml/l) and PTM 1 (8 ml/l). During the induction phase, the following components were added to the bioreactor at a regular interval of 24 hours: casaminoacids at 0.1 % (w/v) (final concentration), Yeast Nitrogen Base at 0.1% (w/v) (final concentration).

*Figure 18* illustrates the evolution of biomass, residual methanol and methanol flow rate, throughout the culture of the recombinant clone of *Pichia pastoris* in a 5 l bioreactor , on a minimal salt medium.

*Figure 19* shows SDS-PAGE 15% analysis of culture supernatant at different induction times of the recombinant clone of *Pichia pastoris* methanol fed batch culture.

### Example 8: Downstream processing steps

### 8.1. Method 1:

At the end of the culture, the medium was centrifuged at 3500 rpm (2000 g), 4°C for 15 minutes. The supernatant was supplemented with Triton X-100 at 1% (final concentration), then further clarified by a microfiltration cartridge (a polysulfone membrane, cut-off equal to 0.1 µm) (Millipore) at a flow rate of 1.5-1.8 l/min.

The IFN alpha containing permeate, was harvested and then desalted using diafiltration or chromatography method.

Diafiltration was carried out using a 5 KDa cartridge (Amersham Biosciences) and lactic acid 50 mM pH4. The retentate was concentrated 10 fold with the same cartridge. All the steps were carried out at room temperature.

Desalting was performed on a column packed with sephadex G25 (Amersham Biosciences). The column was equilibrated with 5 column volumes of lactic acid 50 mM pH4 0.15 M NaCl at a flow rate of 3 cm/min. The supernatant was loaded into the column at a flow rate of 4 cm/min, elution is carried out using 2 column volumes of lactic acid 50 mM pH4 at a flow rate of 3 cm/min.

The concentrated retentate or the desalted supernatant containing IFN alpha, was loaded on Sepharose SP column (Amersham Biosciences, 50 mg protein/ml column material). The column was first washed with 5 column volumes, then activated with 5 column volumes with lactic acid 50 mM pH4, 1M NaCl. The concentrate is loaded into the column, a washing step with the buffer lactic acid 50 mM pH4, is carried out until the OD₂₈₀ becomes equal to zero. Elution of the bound IFN alpha is carried out using 9 column volumes of a stepwise gradient of 0.1- 1 M NaCl, at a flow rate 2.5 cm/min. IFN alpha is eluted from an NaCl concentration equal to 0.4 M to 0.8 M. Fractions containing pure IFN alpha were pooled.

The emergence of proteins in the fractions is monitored by measuring the absorbance at 280 nm. Fractions containing protein were analysed by SDS-PAGE and Western blot.

Interferon α can be further purified on a Sephacryl-S100 column using PBS pH 7.3 at a flow rate of 15 cm/h.

***Figure 20*** illustrates SDS-PAGE 15% analysis of the different fractions collected during the purification of the desalted harvest containing IFNα on Sepharose SP column.

***Figure 21*** illustrates SDS-PAGE 15% analysis of the different fractions collected during the size exclusion purification

### 8.2. Method 2

At the end of the culture, the broth was diluted 4-5 fold with lactic acid 25 mM 0.1 M NaCl pH4 to an OD₆₀₀ equal to 100 (0.3 % (w/v) dry cell weight). The diluted broth is loaded into an expanded bed cation exchange adsorption on STREAMLINE SP XL (Amersham Biosciences).

The flow velocity during expansion/equilibration, adsorption and wash was 300 cm/h, which caused the bed to expand to 3 times during expansion/equilibration and feed application. The buffer used during expansion/equilibration and wash was lactic acid 25 mM pH4. Desorption of the IFN alpha from the adsorbent was performed with downward flow in sedimented mode using lactic acid 25mM pH4 1M NaCl. The flow velocity during the desorption was 100 cm/h.

***Figure* 22** shows Coomassie-blue and silver stained SDS-PAGE 15% gels of the different fractions obtained through the purification of rHuIFNα2b produced at HCD culture on streamline SP XL column.

### Example 9: Analysis of IFN alpha preparations

IFN alpha samples were analysed on 15% SDS polyacrylamide gels under standard reducing conditions. Samples were reduced with mercaptoethanol before electrophoresis. Protein bands were visualized with Coomassie blue staining.

IFN alpha samples were also analysed by SDS electrophoresis followed by silver staining.

The IFN alpha content of various samples obtained during HCD culture and purification was determined by ELISA using monoclonal antibodies and IFNα2b standard produced in-house.

### Example 10: Biological activity of rHuIFNα2b

The biological activity of the rHuIFNα2b preparation was determined by antiviral and Gene Report assays (Meager et al., 2001; Meager 2002).

The antiviral assay is based on the ability of rHuIFNα2b to inhibit the cytopatic effect caused by encephalomyocarditis virus (EMCV) on "glioblastoma cell line" 2D9. Furthermore cell lines (HEK 293P) stably transfected with IFN-inducible promoter sequence (ISRE) linked to SEAP gene (secreted alkaline phosphatase) were used to perform the Gene Report assay. The amount of SEAP in supernatant is proportional to the amount of IFNα in the sample. The SEAP produced is measured using the p-NPP (para-nitrophenyl phosphase) which is a chromogenic substrate for most phosphatases.

The purified was calibrated against the IFNα WHO international standard (code: 95/566). The IFNα has a specific activity varying from ~ 1.57 to 2.85 10⁸ 10⁸IU/mg.

### REFERENCES

Andre, S., Seed, B., Eberle, J., Schraut, W., Bultmann, A. and Haas, J. (1998) Increased immune response elicited by DNA vaccination with a synthetic GP120 sequencewith optimized codon usage. J.Virol 72, 1497-1503.
Apeler, H., Gottschalk, U., Guntermann, D., Hansen, J., Massen, J., Schmidt, E., Schneider, K.H., Schneidereit, M. and Rubsamen-Waigmann, H. (1997) Expression of natural and synthetic genes encoding herpes simplex virus 1 protease in Echerichia coli and purification of the protein. Eur. J. Biochem 247, 890-895.
Babu, K.R., Swaminathan, S., Marten, S., Khanna, N., Rinas, U., 2000. Production of interferon-an in high cell density cultures of recombinant Escherichia coli and its single step purification from refolded inclusion body proteins. Appl. Microbiol. Biotechnol. 53, 655-660
Bagnoli, F. and Lio, P. (1995) Selection, Mutations and codon usage in bacterial model. J. Theoret. Biol 173, 271-281.
Brake, A.J., Merryweather, J.P., Coit, D.G., Heberlein, U.A., Masiarz, F.R., Mullenbach, G.T., Urdea, M.S., Valenzuela, P. and Barr, P.J. (1984) Alpha-factor-directed synthesis and secretion of mature foreign proteins in Saccharomyces cerevisiae. Proc. Natl. Acad. Sci. USA 81, 4642-4646.
Brocca, S., Schmit-Dannert, C., Lotti, M., Alberghinaand, L. and Schmid, R.D. (1998) Design, total synthesis, functional overexpression of the Candida rugosa lip1 gene coding for a major industrial lipase. Protein. Sci 7, 1415-1422.
Bulmer, M. (1987) Coevolution of codons usage and transfer RNA abundance. Nature 325, 728-730.
Cereghino, J.L. and Cregg, J.M., 2000. Heterologous protein expression in the methylotrophic yeast Pichia pastoris. FEMS Microbiol. Reviews. 24, 45-66
Chiruvolu, V., Cregg, J.M. and Meagher, M.M. (1997) Recombinant protein production in an alcohol oxidase-defective strain Pichia pastoris in fed-batch fermentations. Enzyme Microbiol. Technol. 21, 277-283.
Clare, J.J., Rayment, F.B., Ballantine, S.P., Sreekrishna, K. and Romanos, M.A. (1991a) High level expression of tetanus toxin fragment C in Pichia pastoris strains containing multiple tandem integration of gene. Bio/Technology 9, 455-460.
Clare, J.J., Romanos, M.A., Rayment, F.B., Rowedder, J.E., Smith, M.A., Payne, M.M., Sreekrishna, K. and Henwood, C.A. (1991b) Production of mouse epidermal growth factor in yeast: high-level secretion using Pichia pastoris strains containing multiply gene copies. Gene 105, 205-212.
Clare, J.J., Ramanos, M.A., 1995. Expression of cloned genes in the yeast Saccharomyces cerevisiae and Pichia pastoris. Methods Mol. Cell Biol. 5, 319-329
Couderc, R., Baratti, J., 1980. Oxidation of methanol by the yeast Pichia pastoris: purification and properties of alcohol oxidase. Agric. Biol. Chem. 44, 2279-2289
Cregg, J.M., Barringer, K.J., Hessler, A.Y. and Madden, K.R. (1985) Pichia pastoris as a host system for transformation. Mol. Cell. Biol. 5, 3376-3385.
Cregg, J.M., and Madden, K.R. (1987a) Development of yeast transformation systems and construction of methanol-utilization-defective mutants of Pichia pastoris by gene disruption. In: Biological research on Industrial Yeasts (Stewart, G.G., Russel, I., Klein, R.D. and Hiebsch, R.R., Eds.), Vol. 2, pp. 1-18. CRC Press, Boca raton, FL.
Cregg, J.M., Tschopp, J.F., Stilman, C., Siegel, R., Akong, M., Craig, W.S., Buckholz, R.G., Madden, K.R., Kellaris, P.A., Davis, G.R., Smiley, B.L., Cruze, J., Toregrossa, R., Velicelebi, G. and Thill, G.P. (1987b) High-level expression and efficient assembly of hepatitis B surface antigen in methylotrophic yeast, Pichia pastoris. Bio/Technology 5, 479-485.
Cregg, J.M., and Madden, K.R. (1989a) Use of site-specific recombination to generate selectable markers. Mol. Gen. Genet. 219, 320-323.
Cregg, J.M., Madden, K.R., Barringer, K.J., Thill, G.P., Stillman, C.A., (1989b). Functional characterisation of the two alcohol oxidase genes from the yeast Pichia pastoris. Mol. Cell Biol. 9,1316-1323
Grantham, R., Gautier, C. and Gouy, M. (1980) Codon frequencies in 119 individual genes confirm consistent choices of degenerate bases according to genome type. Nucleic. Acids. Res. 8, 1893-1912.
Hasslacher, M., Schall, M., Hayn, M., Bona, R., Rumbold, K., Luckl, J., Griengl, H., Kohlwein, S.D., Schwab, H., 1997. High level intracellular expression of hydroxynitrile lyase from the tropical rubber tree Hevea brasiliensis in microbial hosts. Protein Expr. Purif. 11, 61-71
Higgins, D.R., Busser, K., Comiskey, J., Whittier, P.S., Purcell, T.J., and Hoeffler, J.P. (1998) Small vectors for expression based on dominant drug resistance with direct multicopy selection. Methods Mol. Biol. 103, 41-53.
Hitzeman, R.A., Hagie, F.E., Levine, H.L., Goeddel, D.V., Ammerer, G., Hall, B.D., 1981. Expression of a human gene for interferon in yeast. Nature 293, 717-722
Koutz, P., Davis, G.R., Stillman, C., Barringer, K., Cregg, J. and Thill, G. (1989) Structural comparison of Pichia pastoris alcohol oxidase genes. Yeast 5, 167-177.
Kujan, J. and Herskowitz, I. (1982) Structure of yeast pheromone gene (MF alpha): A putative alpha-factor precursor contains four tandem copies of mature alpha factor. Cell 30, 933-943.
Li, W. and Graur, D. (1991) in: Fundamentals of Molecular Evolution, Sinauer Associates, Sunderland, MA, p91.
Meager, A. (2002) Biological assays for interferons. J.I.M 261, 21-36.
Meager, A., Gaines Das, R., Zoon, K. and Mire-Sluis, A. (2001) Establishment of new and replacement world health organisation international biological standards for human interferon alpha and omega. J.I.M 257, 17-33.
Mizoguchi, J., Pitha, P., Raj, N.B.K., 1985. Efficient expression in E.coli of two species of human interferon-α and their hybrid molecules. DNA 4, 221-32
Nagata, S., Taira, H., Hall, A., Johnsrud, L., Streuli, M., Ecsodi, J., Boll, W., Cantell, K., Weissmann, C., 1980. Synthesis in E. coli of a polypeptide with human leukocyte interferon activity. Nature 284, 316-320
Nagata, T., Uchijima, M., Yochida, A., Kawashima, M. and Koide, Y. (1999) Codon optimization effect on translational efficiency of DNA vaccine in mammalian cells: Analysis of plasmid DNA encoding a STL epitope derived form microorganisms. Biochem. Biophys. Res. Commun 261, 445-451.
Nakamura, Y., Gojobori, T. and Ikemura, T. (1999) Codon usage tabulated from the international DNA sequence data base. Nucleic. Acids. Res 27, 292-292.
Powell, J.R. and Moriyama, E.N. (1997) Evolution of codon usage bias in Drosophilia. Proc. Natl. Acad. Sci. USA 94, 7784-7790.
Remaut, E., Stanssen, P., Fiers, W., 1983. Inducible high level synthesis of mature human fibroblast interferon in E.coli. Nucleic Acids Res. 11, 4677-4688
Romanos, M.A., Clare, J.J., Beesley, K.M., Rayment, F.B., Ballantine, S.P., Makoff, A.J., Dougan, G., Fairweather, N.F. and Charles, I.G. (1991) Recombinant Bordetella pertussis (P69) from Pichia pastoris: High-level production and immunological properties. Vaccine 9, 901-906.
Rossmann, C., Sharp, N., Allen, G., Gewert, D., 1996. Expression and purification of recombinant, glycosylated human interferon alpha 2b in murine Myeloma NSO cells. Protein Exp. Purif. 7, 335-342
Scorer, C.A., Clare, J.J., McCombie, W.R., Romanos, M.A. and Sreekrishna, K. (1994) Rapid selection using G418 of high copy number transformants of Pichia pastoris for high level foreign gene expression. Biotechnology (NY) 12, 181-184.
Sinclair, G. and Choy, F.Y.M. (2002) Synonymous codon usage bias and the expression of Human gluccocerebrosidase in the methylotophic yeast, Pichia pastoris. Protein. Expr. Purif 26, 96-105.
Smith, G.E., Summers, M.D., Fraser, M.J., 1983. Production of human beta interferon in insect cells infected with a baculovirus expression vector. Mol. Cell. Biol. 3, 2156-2165
Sreekrishna, K., Nelles, L., Potenz, R., Cruze, J., Mazzaferro, P., Fish, W., Fuke, M., Holden, K., Phelps, D., Wood, P., Parker, K., 1989. High level expression, purification and characterization of recombinant human tumor necrosis factor synthesized in the methylotrophic yeast Pichia pastoris. Biochemistry 28, 4117-4125
Sreekrishna, K., Barr, K.A., Hoard, S.A., Prevatt, W.D., Torregrosa, R.E., Levingston, R.E., Cruze, J.A. and Wegner, G.H. (1990) Expression of human serum albumin in Pichia pastoris. Topic 09-37B in: Oliver, S.G. and Wickner, R. (Eds), 15th Int. Congr. Yeast Genetics and molecular Biology, Yeast 6 (Special issue). Wieley, New york, NY, p. S447.
Sreekrishna, K. (1993) Strategies for optimization protein expression and secretion in the methylotrophic yeast Pichia pastoris. In: Baltz, R.H., Hegemen, G.D. and Skatrud, P.L. (Eds.), Industrial microorganisms: Basic and Applied Molecular genetics. American Society of Microbiology, Washington, DC, pp. 119-126.
Sreekrishna, K., Prevatt, W.D., Thill, G.P., Davis, G.R., Koutz, P., Barr, K.A. and Hopkins, S.A. (1993) Production of Bacillus entomotoxins in methylotophic yeast. European Patent Application, EP0586 892 A l .
Sreekrishna, K., Brankamp, R.G., Kropp, K.E., Blankenshio, D.T., Tsay, J.T., Smith, P.L., Wierschke, J.D., Subramaniam, A., Birkenberger, L.A., 1997. Strategies for optimal synthesis and secretion of heterologous proteins in the methylotrophic yeast Pichia pastoris. Gene 190, 55-62.
Stratton, J., Chiruvolu, V., Meagher, M., 1998. High cell density fermentation. In: Higgins, D.R., Cregg, J.M., (Eds.) Pichia protocols. Totowa, NJ, Humana Press, pp. 107-121.
Thill, G.P., Davis, G.R., Stillman, S., Holtz, G., Brierly, R., Engel, M., Buckholtz, R., Kenney, J., Provow, S., Vedvick, T. and Siegel, R.S. (1990) positive and negative effects of multicopy integrated expression vectors on protein expression in Pichia pastoris. In. Proceeding of the Sixth International Symposuim on the Genetics of Microorganisms (Penasse, L. Eds.), Vol. 2, pp. 477-490. Société Française de Microbiologie, Paris.
Tschopp, J.F., Sverlow, G., Kosson, R., Craig, W. and Grinna, L. (1987) High level secretion of glycosylated invertase in the methylotrophic yeast, Pichia pastoris. Bio/Technology 5, 1305-1308.
Uchijima, M., Yochida, A., Nagata, T. and Koide, Y. (1998) Optimization of codon usage of plasmid DNA vaccine is required of the effective MHC class I- restricted T cell response against an intacellular bacterium. J. Immun 166, 5594-5599.
Voss, T., Ergulen, E., Ahorn, H., Kubelka, V., Sugylama, K., Maurer-Fogy, I., Glossl, J., 1993. Expression of human interferon omega 1 in SF9 cells. Eur. J. Biochem. 217, 913-919.
Werten, M.W., van den Bosch, T.J., Wind, R.D., Mooibroek, H., de Wolf, F.A., 1999. High yield secretion of recombinant gelatins by Pichia pastoris. Yeast 15, 1087-1096
Zolotukhin, S., Potter, M., Hausworth, W.W, Guyand, J. and Muzyczka, N. (1996) A "humanized" green fluorescent protein cDNA adapted for high-level expression in mammalian cells. J.Virol 70, 4646-4654.
Zsebo, K.M., Lu, H.S., Fiescho, J.C., Goldstein, L., Davis, J., Duker, K., Suggs, S.V., Lai, P.H., Bitter, G.A., 1986. Protein secretion from S. cerevisiae directed by the prepro a factor leader region. J. Biol. Chem. 26, 5858-5865.

## Claims

1. A nucleic acid molecule comprising a coding sequence encoding a human alpha interferon, **characterized in that** said coding sequence is optimized for expression in *Pichia pastoris.*

2. The nucleic acid according to claim 1, wherein said human alpha interferon is HuIFNa2a and said coding sequence is SEQ ID No: 1.

3. An expression vector for transforming a *Pichia pastoris* strain, **characterized in that** it comprises, in the 5' to 3' direction and operably linked :
(i) a *Pichia pastoris-recognized* transcription and translation initiation region,
(ii) a sequence coding for a secretion signal enabling protein secretion in *Pichia pastoris,*
(iii) a coding sequence as defined in claim 1 or claim 2, and
(iv) a *Pichia pastoris*-recognized transcription and translation termination region.

4. A *Pichia pastoris* strain producing human alpha interferon, **characterized in that** it has been transformed by a vector according to claim 3.

5. The *Pichia pastoris* strain according to claim 4, **characterized in that** it has been obtained by transforming a Mut^{S} KM71H *Pichia pastoris* strain.

6. The *Pichia pastoris* strain according to claim 4 or claim 5, **characterized in that** it is IRDF/INF/IPT05, deposited at the *Collection Nationale de Cultures de Microorganismes (CNCM)* on September 11,2006, under the n° I-3668.

7. A nucleic acid comprising a coding sequence encoding a human interferon a2b, wherein said coding sequence is SEQ ID No: 3.

8. An expression vector for transforming a *Pichia pastoris* strain, **characterized in that** it comprises, in the 5' to 3' direction and operably linked,
(i) a *Pichia pastoris*-recognized transcription and translation initiation region,
(ii) a sequence coding for a secretion signal enabling protein secretion in *Pichia pastoris,*
(iii) a coding sequence as defined in claim 8, and
(iv) a *Pichia pastoris-*recognized transcription and translation termination region.

9. A *Pichia pastoris* strain producing human alpha interferon, **characterized in that** it has been transformed by a vector according to claim 8.

10. The *Pichia pastoris* strain according to claim 9, **characterized in that** it has been obtained by transforming a Mut^{S} KM71H *Pichia pastoris* strain.

11. The *Pichia pastoris* strain according to claim 9 or claim 10, **characterized in that** it is KM71H/PICZαrHuIFNα2b, deposited at the *Collection Nationale de Cultures de Microorganismes (CNCM)* on February 01, 2006, under the n° I-3562.

12. A process for producing human alpha interferon by culturing a *Pichia pastoris* strain which expresses and secretes said human alpha interferon, wherein the expression of said interferon is under the control of a promoter regulated by methanol, **characterized in that** it comprises the following steps:
(i) inoculation of a synthetic culture medium in a bioreactor, by a preculture of said *Pichia pastoris* strain, wherein said synthetic medium contains glycerol as a carbon source, and has a defined salt composition;
(ii) batch culture in said bioreactor, at 30°C, pH5, under agitation and oxygenation;
(iii) fed-batch culture on glycerol at 30°C, pH5, under agitation and oxygenation;
(iv) fed-batch culture on methanol, in a synthetic culture medium supplemented with EDTA at a final concentration of 10 mM.

13. The process of claim 12, wherein the *Pichia pastoris* strain is according to any of claims 4-6 and 9-11.

14. The process of claim 12 or claim 13, wherein the synthetic culture medium is obtained as follows:
- glycerol (40 g/l), K₂SO₄ (18.2 g/l), MgSO₄ (7.28 g/l), KOH (4.13 g/l), CaSO₄.2H₂O (0.93 g/l) and 85% orthophosphoric acid (26,7 ml/l) are dissolved in deionised water and sterilized in the bioreactor;
- 5 ml/l basal salts of fermentation PTM1 and 2 ml/l of 500X biotin are added to the medium after sterilization, and culture medium pH is adjusted to 5 by addition of 25% ammonia (w/v), wherein the PTM1 solution contains: CuSO₄.5H₂O (6 g l⁻¹), NaI (0.08 g l⁻¹), MnSO₄.H₂O (3 g l⁻¹), Na₂MoO₄.2H₂O (0.2 g l⁻¹), H₃BO₃ (0.02 g l⁻¹), CoCl₂ (0.5 g l⁻¹), ZnCl₂ (20 g l⁻¹), FeSO₄.7H₂O (65 g l⁻¹), biotin (0.2 g l⁻¹) and H₂SO₄ 98% (5 ml l⁻¹).

15. The process according to any of claims 12 to 14, wherein the fed-batch solution used in step (iii) contains glycerol (450 g/l), PTM1 (8 ml/l) and 500X biotin (5 ml/l) and is added at a rate of 15 ml/l/h.

16. The process according to any of claims 12 to 15, wherein the fed-batch solution used in step (iv) contains methanol at 100% (973 ml/l), 500X biotin (5 ml/l) and PTM1 (8 ml/l) and the feeding rate is modulated to keep the methanol residual level under 0.6% (v/v).

17. The process according to any of claims 12 to 16, wherein the induction phase lasts for at least three days, preferably 4 or 5 days, during which the following components are added to the bioreactor at regular intervals of 24 hours: casaminoacids at a final concentration of 0.1% (w/v) and Yeast Nitrogen Base at a final concentration of 0.1% (w/v).

18. The process according to any of claims 12 to 17, wherein step (iii) is initiated once the optical density at 600 nm of the culture of step (ii) has reached 80, and wherein the fed-batch culture of step (iii) lasts until the dry weight reaches at least 100 g/l, preferably 110-120 g/l.

19. The process according to any of claims 12 to 18, wherein between steps (iii) and (iv), the cells are harvested, resuspended in fresh medium and returned back to the bioreactor.

20. The process according to any of claims 12 to 19, wherein the preculture used in step (i) is in BMGY medium.

21. A process for purifying human alpha interferon secreted in the culture medium by recombinant yeasts, **characterized in that** it comprises the following steps:
(i) eliminating the biomass by centrifugation,
(ii) filtering the supernatant through a 0.1 µm cartridge in the presence of Triton X-100 at 1%,
(iii) desalting the permeate by diafiltration using a 5 kDa cartridge, and
(iv) purifying the supernatant by a cation exchange chromatography.

22. The process of claim 21, wherein the desalting step (iii) is performed by chromatography on a Sephadex G25 column.

23. A process for purifying human alpha interferon secreted in the culture medium by recombinant yeasts, **characterized in that** it comprises the following steps:
(i) dilution of the culture medium with a solution of lactic acid (25 mM), NaCl (0.1 M), pH4, to an optical density at 600 nm equal to 100; and
(ii) purification of the diluted medium using an expanded bed chromatography on a cation exchange support.

24. The process according to any of claims 21 to 23, comprising an additional step of purification by gel filtration using sephacryl S-100.

25. The process according to any of claims 21 to 24, wherein said human alpha interferon has been produced by a recombinant *Pichia pastoris* strain according to any of claims 4-6 and 9-11.

26. The process according to any of claims 21 to 25, wherein said human alpha interferon has been produced by a process according to any of claims 12 to 20.


SEQUENCE LISTING

<110> INSTITUT PASTEUR
INSTITUT PASTEUR DE TUNIS

<120> Recombinant Pichia pastoris strains and process for the
production of recombinant human interferon alpha

<130> VMAmad226/124EP1

<160> 17

<170> PatentIn version 3.3

<210> 1
<211> 498
<212> DNA
<213> Artificial

<220>
<223> Synthetic rHu IFNalpha2a (AAA/23) cDNA sequence

<400> 1
tgtgatttgc ctcaaaccca ctccttgggt tccagaagaa ccttgatgtt gttggcacag 60

atgagaaaaa tctctttgtt ctcctgcttg aaggacagac atgactttgg atttccacag 120

gaggagtttg gcaaccagtt ccaaaaggct gaaaccatcc ctgtcttgca tgagatgatc 180

cagcagatct tcaatttgtt ctccacaaag gactcttctg ctgcttggga tgagaccttg 240

ttggacaaat tctacactga attgtaccag cagttgaatg acttggaagc ctgtgttatc 300

cagggtgttg gtgttacaga gactccattg atgaaggagg actccatttt ggctgttaga 360

aaatacttcc aaagaatcac tttgtatttg aaagagaaga aatactcccc ttgtgcctgg 420

gaggttgtca gagcagaaat catgagatct ttttctttgt caacaaactt gcaagaaagt 480

ttaagaagta aggaatga 498

<210> 2
<211> 165
<212> PRT
<213> Artificial

<220>
<223> Synthetic rHuIFNalpha2a (K/23) Amino acid sequence

<400> 2

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165

<210> 3
<211> 498
<212> DNA
<213> Artificial

<220>
<223> rHuIFNalpha2b (AGA/23) cDNA inframe Sequence

<400> 3
tgtgatctgc ctcaaaccca cagcctgggt agcaggagga ccttgatgct cctggcacag 60

atgaggagaa tctctctttt ctcctgcttg aaggacagac atgactttgg atttccccag 120

gaggagtttg gcaaccagtt ccaaaaggct gaaaccatcc ctgtcctcca tgagatgatc 180

cagcagatct tcaatctctt cagcacaaag gactcatctg ctgcttggga tgagaccctc 240

ctagacaaat tctacactga actctaccag cagctgaatg acctggaagc ctgtgtgata 300

cagggggtgg gggtgacaga gactcccctg atgaaggagg actccattct ggctgtgagg 360

aaatacttcc aaagaatcac tctctatctg aaagagaaga aatacagccc ttgtgcctgg 420

gaggttgtca gagcagaaat catgagatct ttttctttgt caacaaactt gcaagaaagt 480

ttaagaagta aggaatga 498

<210> 4
<211> 165
<212> PRT
<213> Homo sapiens

<220>
<221> misc_feature
<223> Human IFNalpha2b (R/23) Amino acid sequence

<400> 4


<210> 5
<211> 28
<212> DNA
<213> Artificial

<220>
<223> FIFNalpha 2b primer

<400> 5
tggaattctg tgatctgcct caaaccca                                        28


<210> 6
<211> 37
<212> DNA
<213> Artificial

<220>
<223> RIFNalpha 2b primer

<400> 6
attctgcggc cgctcattcc ttacttctta aactttc                    37


<210> 7
<211> 11
<212> PRT
<213> Artificial

<220>
<223> C-terminal myc epitop tag

<400> 7

Glu Gln Lys Leu Ile Ser Glu Glu Asp Leu Asn
1               5                   10


<210> 8
<211> 92
<212> DNA
<213> Artificial

<220>
<223> synthetic primer Fa1

<400> 8
tggaattctg tgatttgcct caaacccact ccttgggttc cagaagaacc ttgatgttgt     60

tggcacagat gagaaaaatc tctttgttct cc                                   92


<210> 9
<211> 60
<212> DNA
<213> Artificial

<220>
<223> Synthetic primer Fb1

<400> 9
aaactcctcc tgtggaaatc caaagtcatg tctgtccttc aagcaggaga acaaagagat     60


<210> 10
<211> 78
<212> DNA
<213> Artificial

<220>
<223> synthetic primer Fc1

<400> 10
ccacaggagg agtttggcaa ccagttccaa aaggctgaaa ccatccctgt cttgcatgag     60

atgatccagc agatcttc                                                   78


<210> 11
<211> 22

<212>  DNA
<213>  Artificial

<220>
<223>  Ffgsl primer

<400>  11
tggaattctg tgatttgcct ca                                        22


<210>  12
<211>  21
<212>  DNA
<213>  Artificial

<220>
<223>  Rfgsl primer

<400>  12
gaagatctgc tggatcatct c                                         21


<210>  13
<211>  102
<212>  DNA
<213>  Artificial

<220>
<223>  Fa2 primer

<400>  13
cagatcttca atttgttctc ctccacaaag gactcttctg ctgcttggga tgagaccttg     60

ttggacaaat tctacactga attgtaccag cagttgaatg ac                        102


<210>  14
<211>  99
<212>  DNA
<213>  Artificial

<220>
<223>  Fb2 primer

<400>  14
gtattttcta acagccaaaa tggagtcctc cttcatcaat ggagtctctg taacaccaac     60

accctggata acacaggctt ccaagtcatt caactgctg                            99


<210>  15
<211>  99
<212>  DNA
<213>  Artificial

<220>
<223>  Fc2 primer

<400>  15

gctgttagaa aatacttcca aagaatcact ttgtatttga aagagaagaa atactcccct     60

tgtgcctggg aggttgtcag agcagaaatc atgagatct                           99

<210>  16
<211>  25
<212>  DNA
<213>  Artificial

<220>
<223>  FfgsII primer

<400>  16
tgaagatctt caatttgttc tccac                                          25

<210>  17
<211>  26
<212>  DNA
<213>  Artificial

<220>
<223>  RfgsII primer

<400>  17
tgaagatctc atgatttctg ctctga                                         26